# EUROPEAN PATENT SPECIFICATION

(11) **EP 0 694 044 B1**
(45) Date of publication and mention of the grant of the patent: **22.03.2006**
(21) Application number: 94915385.2
(22) Date of filing: 15.04.1994
(51) Int. Cl.: C12N 9/12, C07K 16/40, C07H 21/04, A61K 38/43

(54) **METHOD AND PRODUCT FOR REGULATING CELL RESPONSIVENESS TO EXTERNAL SIGNALS**
VERFAHREN UND PRODUKT FÜR DIE REGULATION DES REAKTIONSVERMÖGENS VON ZELLEN AUF ÄUSSERE SIGNALE
PROCEDE ET PRODUIT DE REGULATION DE LA REACTIVITE CELLULAIRE A DES SIGNAUX EXTERNES

(30) Priority: 15.04.1993 US 49254
(43) Date of publication of application: 31.01.1996
(73) Proprietor: NATIONAL JEWISH CENTER FOR IMMUNOLOGY AND RESPIRATORY MEDICINE, Denver, CO 80206 (US)
(72) Inventor: JOHNSON, Gary L., Boulder, CO 80304 (US)
(74) Representative: Kirkham, Nicholas Andrew
(86) International application number: PCT/US1994/004178
(87) International publication number: WO 1994/024159

(56) References cited:
- Molecular and Cellular Biology, Volume 5, Number 8, issued August 1985, CHALEFF et al., "Molecular Cloning and Characterization of the STE7 and STE11 Genes of Saccharomyces Cerevisiae", pages 1878-1886, see pages 1880-1885.
- Science, Volume 258, issued 16 October 1992, CREWS et al., "The Primary Structure of MEK, a Protein Kinase that Phosphorylates the ERK Gene Product", pages 478-480, see page 479.
- Science, Volume 257, issued 04 September 1992, DENT et al., "Activation of Mitogen-Activated Protein Kinase Kinase by v-RAF in NIH 3T3 Cells and in Vitro", pages 1404-1407, see pages 1404 and 1406.
- Nature, Vol. 358, issued 30 July 1992, KYRIAKIS et al., "Raf-1 Activates MAP Kinase-Kinase", pages 417-421, see pages 419 and 421.
- Science, Volume 260, issued 16 April 1993, LANGE-CARTER et al., "A Divergence in the MAP Kinase Regulatory Network Defined by MEK Kinase and Raf", pages 315-319, see pages 316 and 317.
- Molecular and Cellular Biology, Volume 11, Number 7, issued July 1991, WANG et al., "Byr2, a Schizosaccharomyces Pombe Gene Encoding a Protein Kinase Capable of Partial Suppression of the RasI Mutant Phenotype", pages 3554-3563, see pages 3556-3561.
- CHALEFF ET AL: 'Molecular Cloning and Characterization of the STE7 and STE11 Genes of Saccharomyces Cerevisiae' MOLECULAR AND CELLULAR BIOLOGY vol. 5, no. 8, August 1985, pages 1880 - 1885

## Description

### FIELD OF THE INVENTION

This invention relates to isolated MEK kinase nucleic acid sequences, substantially pure MEKK proteins and methods involved in the regulation and use of MEKK, MEK and MAPK enzymes.

### BACKGROUND OF THE INVENTION

Mitogen-activated protein kinase (MAPKs) (also called extracellular signal-regulated kinases or ERKs) are rapidly activated in response to ligand binding by both growth factor receptors that are tyrosine kinases (such as the epidermal growth factor (EGF) receptor) and receptors that are coupled to heterotrimeric guanine nucleotide binding proteins (G proteins) such as the thrombin receptor. The MAPKs appear to integrate multiple intracellular signals transmitted by various second messengers. MAPKs phosphorylate and regulate the activity of enzymes and transcription factors including the EGF receptor, Rsk 90, phospholipase A₂, arachidonic acid metabolites, c-Myc, and perhaps c-Jun. Although the rapid activation of MAPKs by receptors that are tyrosine kinases is dependent on Ras, G protein-mediated activation of MAPK appears to occur predominantly through pathways independent of Ras.

Complementation analysis of the pheromone-induced signaling pathway in yeast has defined a protein kinase system that controls the activity of Spkl and Fus3-Kssl, the *Schizosaccharomyces pombe* and *Saccharomyces cerevisiae* homologs of MAPK (see for example, B.R. cairns et al., *Genes and Dev.* 6, 1305 (1992) ; B.J. Stevenson et al., *Genes and Dev.* 6, 1293 (1992); S.A. Nadin-Davis et al., *EMBO J.* 7, 985 (1988); Y. Wang et al., *Mol. Cell. Biol.* 11, 3554 (1991). In *S. cerevisiae*, the protein kinase Ste7 is the upstream regulator of Pus3-Kss1 activity; the protein kinase Stell regulates Ste7. The *S. pombe* gene products Byr1 and Byr2 are homologous to Ste7 and Stell, respactively. The MEK (MAPK Kinase or ERK Kinase) or MKK (MAP Kinase kinase) enzymes are similar in sequence to ste7 and Byr1. The MEKs phosphorylate MAPKs on both tyrosine and threonine residues which results in activation of MAPK. The mammalian serine-threonine protein kinase Raf phosphorylates and activates MEK, which leads to activation of MAPK. Raf is activated in response to growth factor receptor tyrosine kinase activity and therefore Raf may activate MAPK in response to stimulation of membrane-associated tyrosine kinases. Raf is unrelated in sequence to *Stel1* and *Byr2*. Thus, Raf may represent a divergence in mammalian cells from the pheromone-responsive protein kinase system defined in yeast. Cell and receptor specific differences in the regulation of MaPKs suggest that other Raf independent regulators of mammalian MEKs exist.

### SUMMARY OF THE INVENTION

In one aspect the invention relates to a substantially pure protein selected from the group consisting of: (a) comprising the amino acid sequence as set forth in SEQ ID NO 4 or a kinase catalytic domain or regulatory domain thereof; or (b) comprising the amino acid sequence as set forth in SEQ ID No 5, or a kinase catalytic domain or regulatory domain thereof.

In another aspect the invention relates to a substantially pure protein comprising an amino acid sequence at least 80% identical to a MEKK amino acid sequence set forth in SEQ ID NO 4, or SEQ ID NO 5.

Preferred aspects of the protein are defined in claims 3 and 4.

In another aspect the invention relates to a substantially pure protein comprising a kinase domain amino acid sequence at least 80% identical to a kinase domain amino acid sequence set forth in SEQ ID NO 8 or SEQ ID NO 9.

Preferred aspects of the protein are defined in claims 6 and 7.

In another aspect the invention relates to an antibody that specifically binds to these proteins.

ln another aspect the invention relates to a method to identify compounds capable of regulating signals initiated from a receptor on the surface of a cell comprising:
(a) contacting a cell containing a protein of the invention with a putative regulatory compound;
(b) contacting said cell with a ligand capable of binding to said receptor; and
(c) assessing the ability of said putative regulatory compound to regulate said signals by determining the activity of said protein.

In another aspect the invention relates to a method to identify compounds capable of regulating signal transduction in a cell comprising:
(a) contacting a putative inhibitory compound with the protein of the invention to form a reaction mixture;
(b) contacting the reaction mixture with MEKK protein; and
(c) assessing the ability of the putative inhibitory compound to inhibit the activity of said MEKK protein.

The present invention is directed to a novel gene, denoted MEKK or mammalian MEK kinase. The regulation of MEKK is useful in various therapeutic applications that involve differentiation and mitogenesis. In particular, the present invention is directed to the regulation of mitogen-activated protein kinases (MAPKs) which the present inventor has discovered is activated by MEKK. Because MAPKs are known to phosphorylate and regulate the activity of enzymes and/or transcription factors, the present invention is directed to the regulation of such activities either through manipulation of the nucleic acid sequences encoding MEKK and/or through administration of MEKK to desired cell populations. The present invention is also directed to the dual regulation by Raf and MEKK to effect desired levels of MAPK activity and to alter typical responses to ligand binding by both growth factor receptors, such as tyrosine kinases, and receptors that are coupled to G proteins, such as the thrombin receptor. The present invention is therefore directed to substantially pure MEKK, to isolated nucleic acid sequences encoding MEKK, to host cells transformed with the MEKK gene, and to antibodies to the MEKK protein. The present invention is further directed to gene therapy using the MEKK gene to regulate responses to ligand binding by growth factor receptors and G protein-linked receptors.

One aspect of the present invention involves the inactivation of MEKK to inhibit metastasis, various forms of cancer, autoimmune diseases, allergic responses and inflammatory responses. Another aspect involves the stimulation of MEKK production to facilitate wound healing activities. The present invention is therefore useful in controlling the activity of neutrophil, macrophage and basophil cells in inflammatory responses in the lung and various other tissues. The present invention is useful in affecting pathways regulated by platelet activating factors, and thrombin receptors. It also finds application in the inhibition of angiogenesis and certain kinds of tumors, particularly lung carcinomas and other epithelial carcinomas and the treatment of smooth muscle and arterial tissues. Additionally, the present invention can be used to inhibit chemotactic responses of neutrophils and macrophages, as well as to ameliorate allergic responses.

The present invention also encompasses the use of the *MEKK* gene in gene therapy whereby stimulation of differentiation and mitogenesis can be achieved to, for example, alleviate atrophy of certain cell types, such as in Parkinson's disease or Alzheimer's disease by acting as a neurotropic growth factor.

In another embodiment, MEKK is used as a screen for oncogenes and tumor agents by using a screening assay to determine whether such proteins are activated by MEKK. Antibodies made against MEKK can be used to determine the changes in levels of expression of MEKK that might be involved in certain cancers and autoimmune diseases.

The present inventor has recognized that when ligands are bound to B cell or T cell surface receptors, MEKK is a predominant phosphorylated product resulting from the ligation of the receptor. As such MEKK is believed to be a major regulatory protein in the signaling pathway of T and B cells. The regulation of MEKK expression is therefore believed to be useful in the regulation of the growth and differentiation of T and B cells.

MEKK can be used to produce antibodies for use in diagnostic and therapeutic applications. For instance, antibodies against MEKK can be used to screen for tumor cells expressing oncoproteins that are activated by MEKK.

In another embodiment of the invention, the MEKK protein can be used for stimulation of cell growth, mitogenesis and differentiation in vitro. For example, any particular population of cells that one desires to expand and differentiate may be achieved by exposing such cells to effective amounts of MEKK.

In yet another embodiment of the invention, MEKK protein can be provided in a suitable formulation for administration to desired cells to effect an antiinflammatory response.

In another embodiment of the invention, host cells are transformed with the MEKK gene. The MEKK gene is therefore useful as a tool in gene therapy for the treatment of various diseases, including cancer, autoimmune diseases, neuronal diseases, muscular diseases, allergic responses and inflammatory responses. As used herein, "gene therapy" refers to delivery of a desired gene to a particular cell population to effect expression of that gene in such cell population.

### BRIEF DESCRIPTION OF THE DRAWINGS

Fig. 1A is a Northern (RNA) blot of a single 7.8 kb MEKK mRNA in several cell lines and mouse tissues.
Fig. 1B shows a Southern (DNA) blot of the MEKK gene.
Fig. 1C is an immunoblot showing expression of the 78-kd and 50-kd forms of MEKK in rodent cell lines.
Fig. 2A shows the activation of MAPK in COS cells transfected with MEKK.
Fig. 2B is an immunoblot showing expression of MEKK in cells transfected with vector only (control) or vector encoding MEKK that were treated with or without EGF.
Fig. 3 shows the activation and phosphorylation of MEK in COS cells transfected with MEKK.
Fig. 4A shows the phosphorylation of MEK-1 by MEKK.
Fig. 4B shows the time course of phosphorylation of MEK-1 by MEKK expressed in COS cells.
Fig. 4C is an immunoblot of MEKK overexpressed in COS cells.
Fig. 5A shows the phosphorylation of MAPK by activated MEK-1.
Fig. 5B shows phosphorylation of MEK-1 by immunoprecipitated MEKK.
Fig. 6A shows the phosphorylation of MEK-1 by activated Raf.
Fig. 6B shows the activity of Raf in COS cells overexpressing MEKK treated with EGF.
Fig. 7 shows immunprecipitates of MEKK protein using MEKK antiserum.
Fig. 8 shows the phosphorylation of kinase inactive MEK-1 by immunoprecipitated MEKK or Raf-B.
Fig. 9 shows a time course of EGF-stimulated MEKK and Raf-B activation.
Fig. 10 shows immunodepletion of Raf-B from MEKK immunoprecipitates.
Fig. 11 shows immunodepletion of Raf-B decreases Raf-B activity.
Fig. 12 shows inhibition of MEKK and Raf-B activation by dominant negative N¹⁷RAS expression.
Fig. 13 shows inhibition of EGF activation of MEKK by forskolin.
Fig. 14 is a schematic representation of the signal pathways of vertebrates and yeast.
Fig. 15 illustrates improved MEKK activity by truncated MEKK molecules.

### DETAILED DESCRIPTION OF THE INVENTION

The present invention relates to a novel MEKK protein capable of regulating signal transduction in cells. An MEKK protein of the present invention can include a full-length MEKK protein or a portion of an MEKK protein. An MEKK protein of the present invention is capable of phosphorylating MEK protein and/or inhibiting MEKK kinase activity. Preferably, an MEKK protein of the present invention comprises at least a portion of an MEKK catalytic domain such that the protein is capable of phosphorylating MEK protein, and/or at least a portion of an MEKK regulatory domain such that the protein is capable of inhibiting MEKK kinase activity.

*Stel1* and *Byr2* nucleic acid sequences were used to identify mammalian MEK kinase (MEKK) cDNA. Unique degenerate inosine oligodeoxynucleotides were designed to correspond to regions of sequence identity between the yeast *Stel1* and *Byr2* genes. With primers and cDNA templates derived from polyadenylated RNA from NIH 3T3 cells, a polymerase chain reaction (PCR) amplification product of 320 base pairs (bp) was isolated. This 320 bp cDNA was used as a probe to identify a MEKK cDNA (herein referred to as MEKK1) of 3260 bp from a mouse brain cDNA library. The MEKK 1 nucleotide sequence was determined by dideoxynucleotide sequencing of double-stranded DNA. The nucleic acid sequence and translated amino acid sequence for MEKK1 are shown in Table 1.

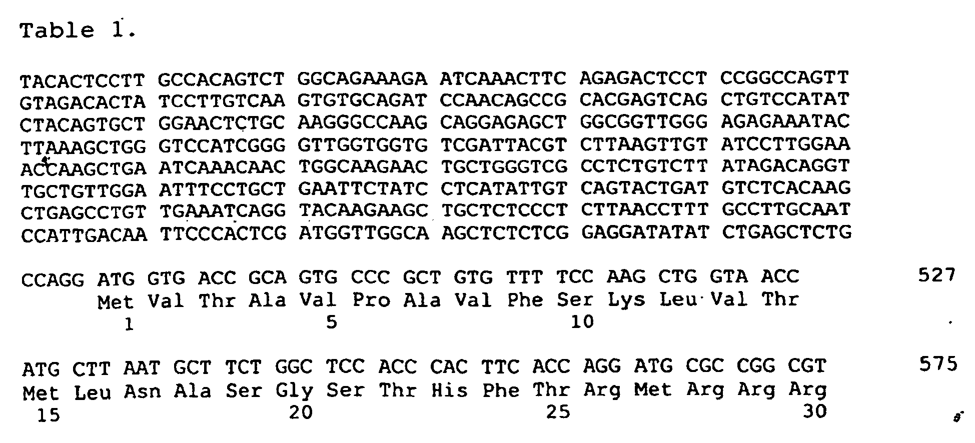

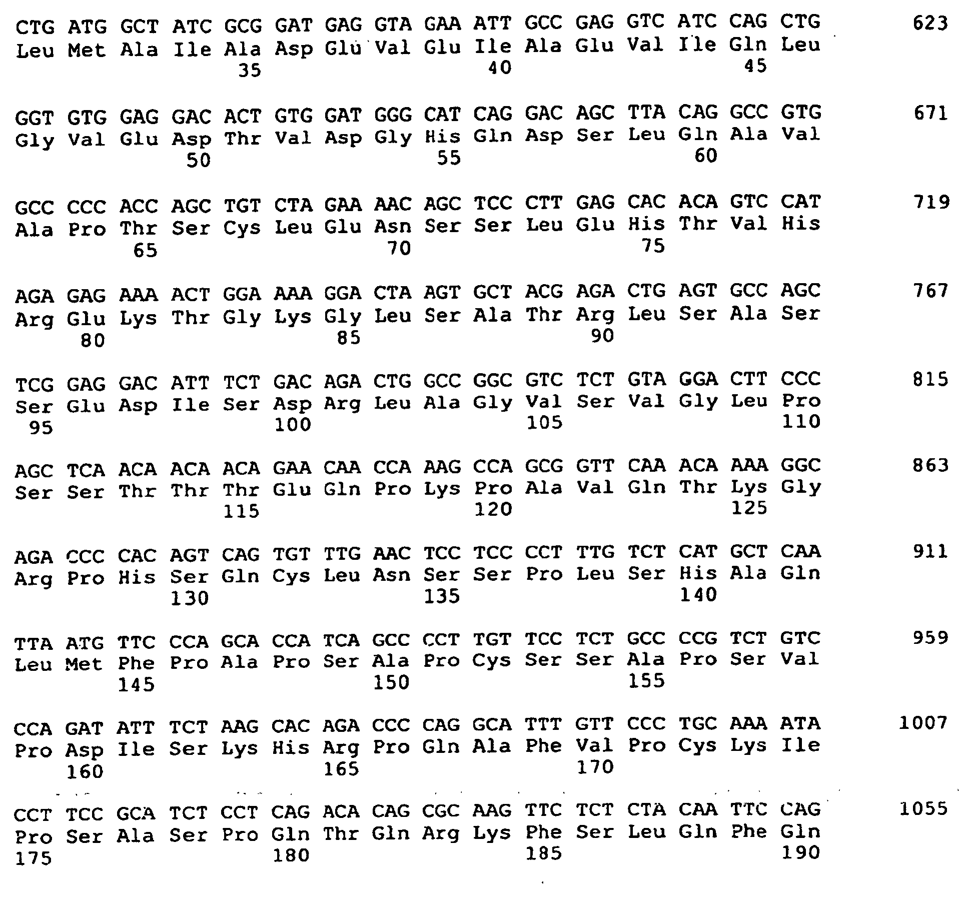

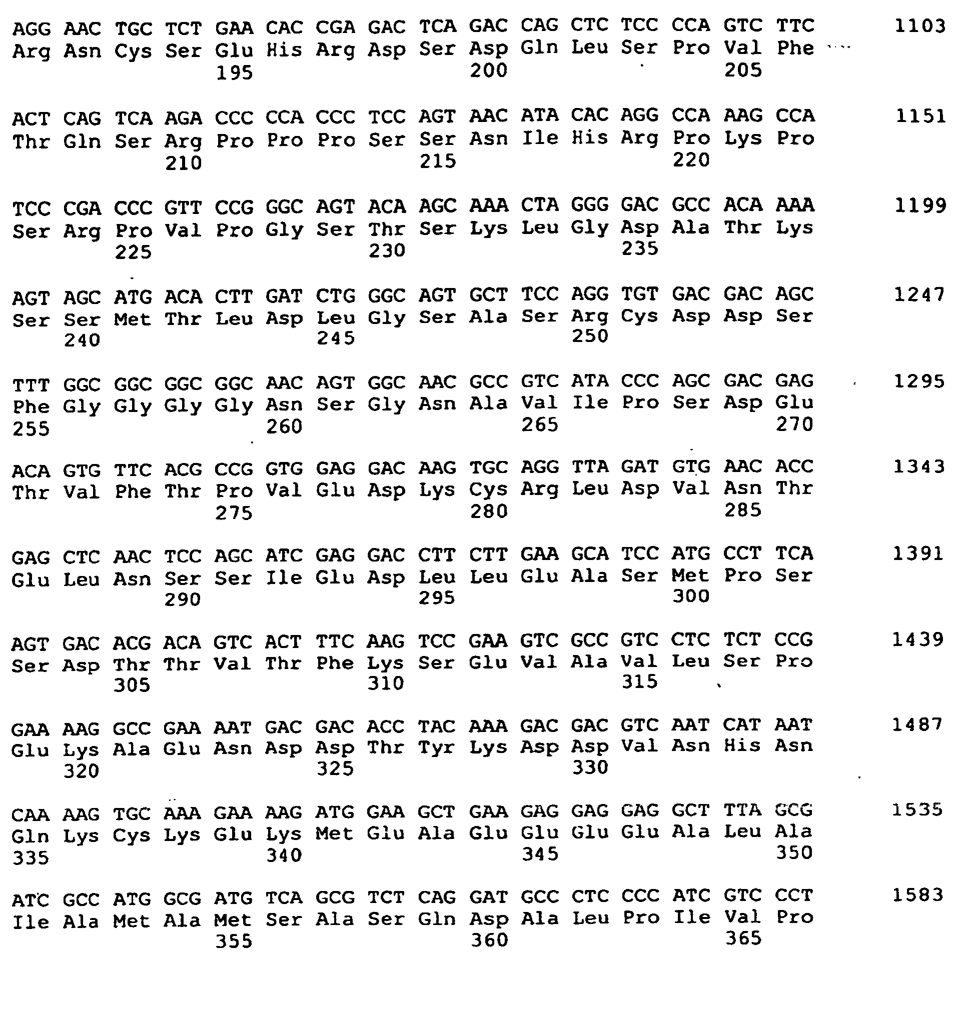

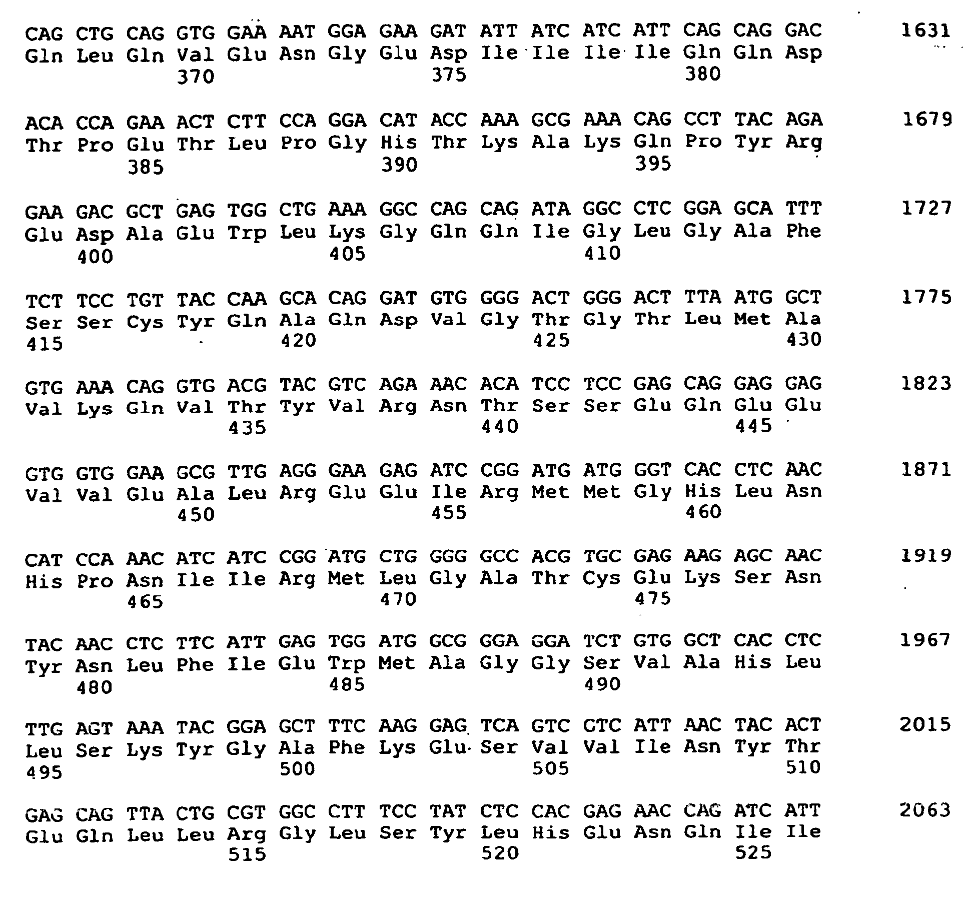

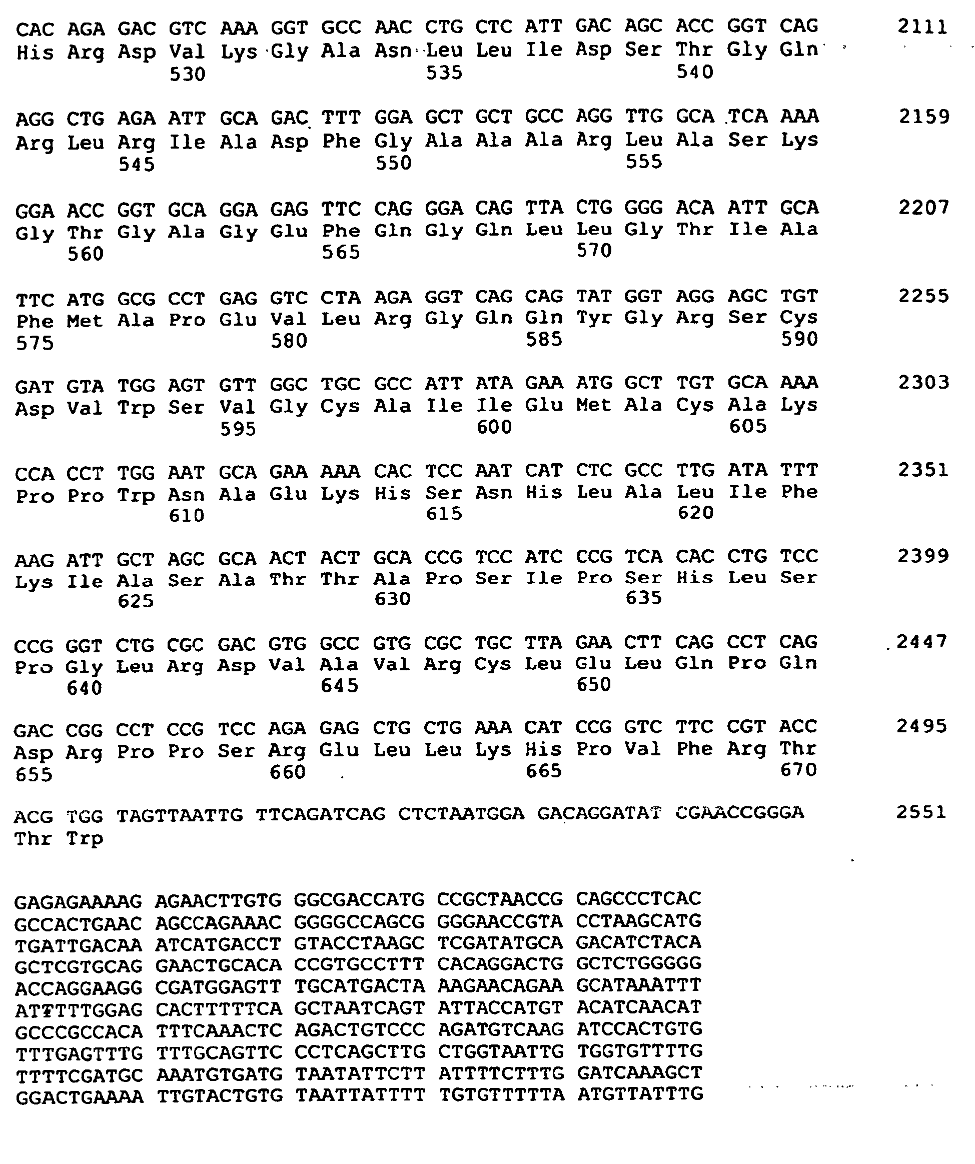

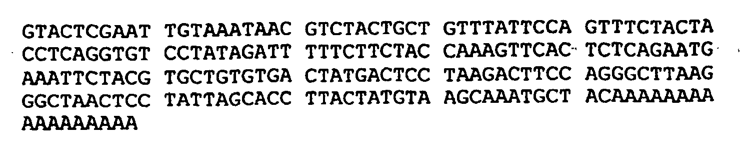

Additional unique MEKK proteins, MEKK2, MEKK3 and MEKK 4, were identified by PCR. The amino acid sequences for MEKK 2 and MEKK 3 compared with the amino acid sequence of MEKK 1 are shown in Table 2.

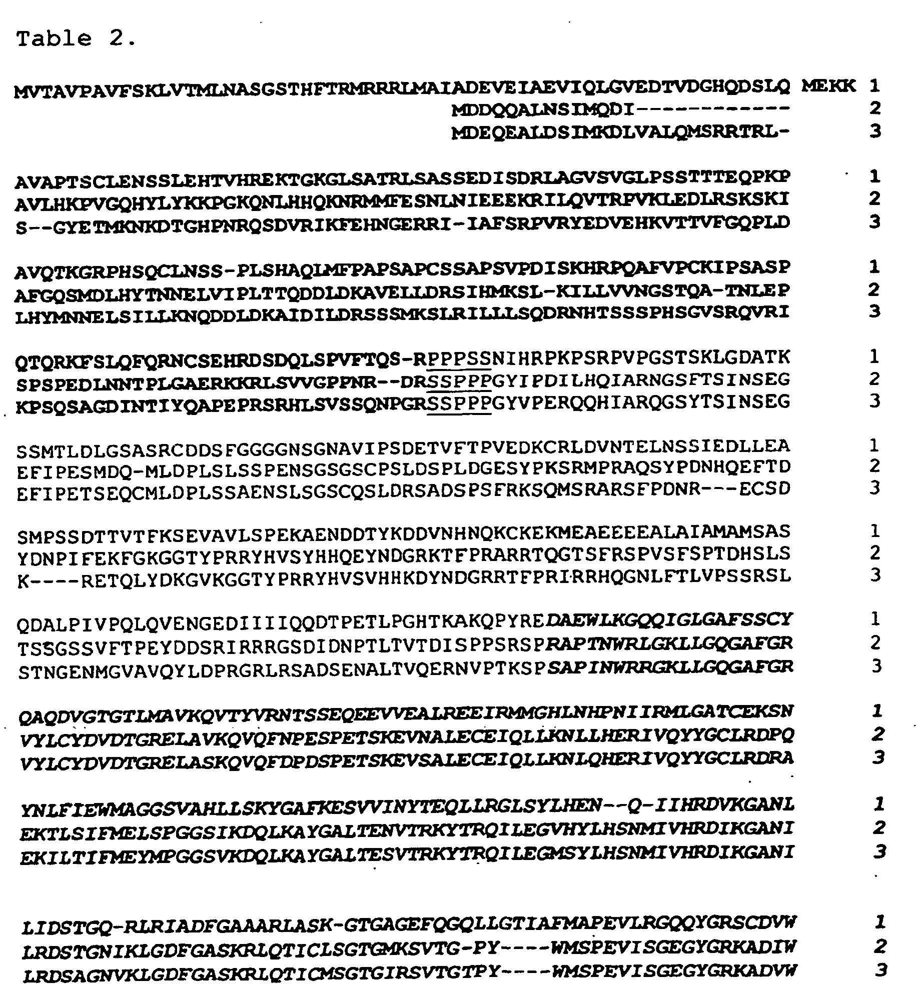

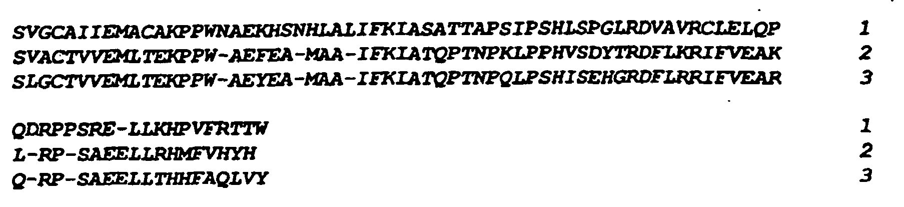

**Bold Amino Terminus- Regulatory Domain**
Underline sequence- Regulatory hinge Sequence
***Bold Italics- Catalytic Domain*** The amino acid sequence of the kinase domain of MEKK 4 is shown in Table 3 compared with the kinase domains of MEKK 1, MEKK 2 and MEKK 3.

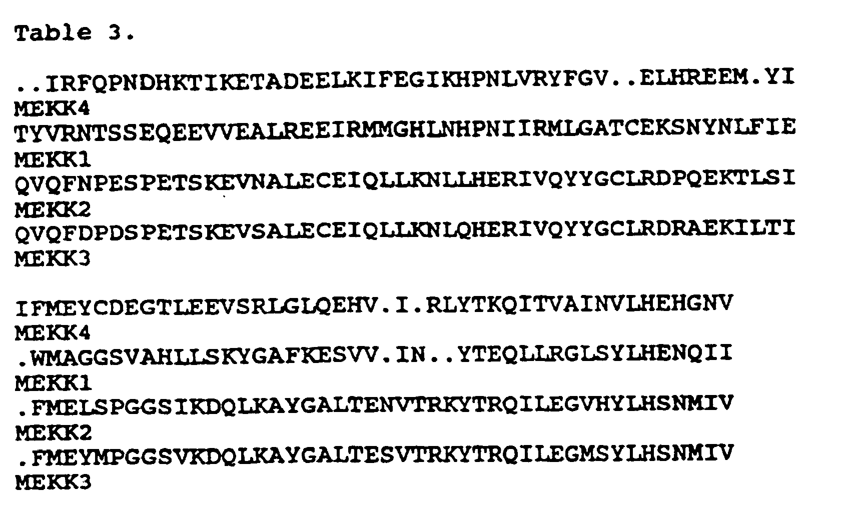

Referring to Table 1, based on the Kozak consensus sequence for initiation codons, the starting methionine can be predicted to occur at nucleotide 486. With this methionine at the start, the cDNA encodes a protein of 672 amino acids, corresponding to a molecular size of 73 kD. There is another in-frame methionine at position 441, which does not follow the Kozak rule, but would yield a protein of 687 amino acid residues (74.6 kD). This size range correlates with the apparent molecular size of 78 to 80 kD of MEKK determined by SDS-polyacrylamide gel electrophoresis (PAGE) and immunoblotting.

The primary sequence of the MEKK protein suggests two functional domains, (i) an NH₂-terminal moiety rich in serine and threonine that may serve a regulatory role and (ii) a COOH-terminal protein kinase catalytic domain. Twenty percent of the NH₂-terminal 400 amino acids are serine or threonine whereas there are only two tyrosines. Several potential sites of phosphorylation by protein kinase C are apparent in the NH₂-terminal region; no SH2 or SH3 domains are encoded in the MEKK sequence. The catalytic domain is located in the COOH-terminal half of MEKK .

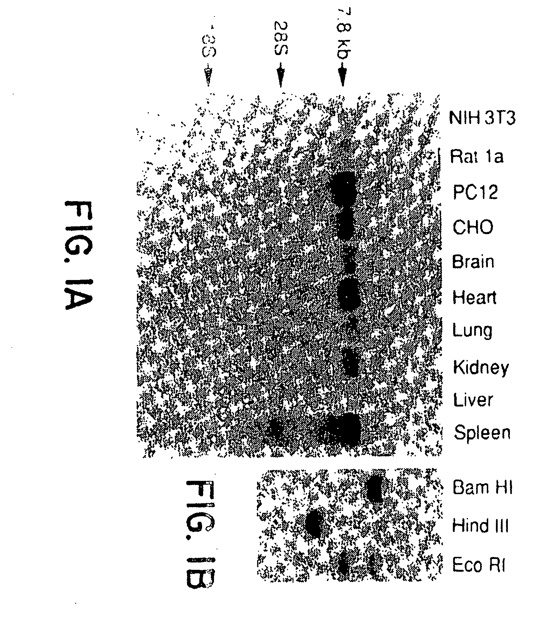

MEKK is encoded by a 7.8 kb mRNA that is expressed in several cell lines and mouse tissues. Fig. 1A shows a Northern (RNA) blot of a single 7.8-kb MEKK mRNA in several cell lines and mouse tissues. Equal amounts (20 *µ*g) of total RNA were loaded onto the gel as indicated by ethidium bromide staining. Blots were probed with either a 320-bp cDNA fragment encoding a portion of the MEKK kinase domain or an 858-bp fragment encoding a portion of the NH₂ terminal region of MEKK. The MEKK mRNA is highly expressed in mouse heart and spleen, whereas low amounts are present in liver. The 7.8 kb MEKK mRNA was identified with probes derived from both the 5' and 3' ends of the MEKK cDNA. Thus, the MEKK cDNA is missing a putative untranslated sequence of about 4 kb.

MEKK is believed to be the product of a single gene. Fig. 1B shows a Southern (DNA) blot of MEKK gene. Mouse genomic DNA (10 *µ*g) was digested with either *Bam HI, Hind III* or *Eco RI* and applied to gels. Blots were probed with a 320-bp fragment of the *MEKK* gene. The appearance of one band in the *Bam HI* and *Hind III* digests suggests that MEKK is encoded by one gene. The appearance of two bands in the *Eco RI* digest indicates the likely presence of an *Eco RI* site within an intron sequence spanned by the probe. The MEKK mRNA is highly expressed in mouse heart and spleen, whereas low amounts are present in liver. The 7.8 kb MEKK mRNA was identified with probes derived from both the 5' and 3' ends of the MEKK cDNA. Thus, the MEKK cDNA is missing a putative untranslated sequence of about 4 kb.

Immunoblots of cell lysates probed with affinity-purified antibodies to the 15-amino acid peptide DRPPSRELLKHPVER derived from the COOH-terminus of MEKK defined a prominent band migrating at 78 kD.

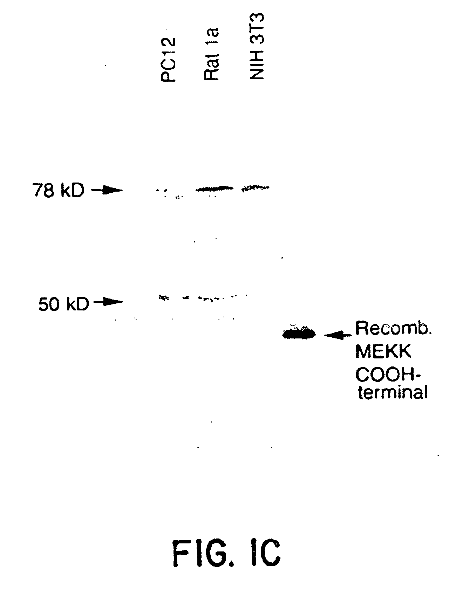

Fig. 1C shows an immunoblot of expression of the 78-kD and 50-kD forms of MEKK in rodent cell lines. Soluble cellular protein (100 *µ*g) or recombinant MEKK COOH-terminal fusion protein (30 ng) was loaded onto the gel for immunoblotting with affinity-purified MEKK antibody (1:300 dilution). Pheochromocytoma (PC12), Rat la, and NIH 3T3 cells contained the same 78-kD immunoreactive protein, which often migrated as a doublet on SDS-PAGE. A prominent 50-kD immunoreactive species was also commonly present but varied in intensity from preparation to preparation. It is believed to be a proteolytic fragment of the 78-kD protein. Visualization of both the 78- and 50-kD immunoreactive bands on immunoblots was inhibited by incubation of the 15-amino acid peptide antigen with the antibody. The MEKK protein detected by immunoblotting is similar to the molecular size predicted from the open reading frame of the *MEKK* cDNA.

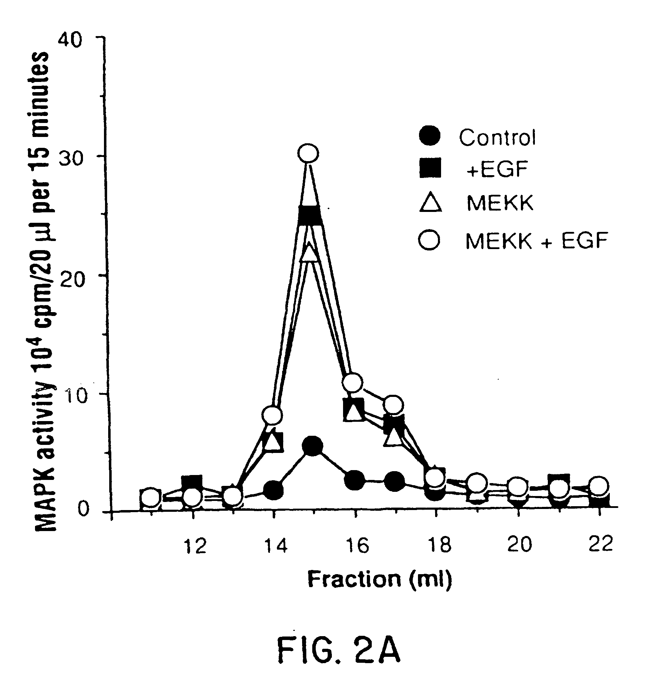

The MEKK protein was expressed in COS-1 cells to define its function in regulating the signaling system that includes MAPK. Referring to Fig. 2A, when MEKK was overexpressed in COS-1 cells, MAPK activity was four to five times greater than that in control cells transfected with plasmid lacking a *MEKK* cDNA insert. COS cells in 100-mm culture dishes were transfected with either the pCVMV5 expression vector alone (1 *µ*g: control) or the pCVMV5 MEKK construct (1 *µ*g: MEKK). After 48 hours, the cells were placed in serum-free medium containing bovine serum albumin (0.1 percent) for 16 to 18 hours to induce quiescence. Cells were then treated with human EGF (30 ng/ml) (+EGF) or buffer (control) for 10 minutes, washed twice in cold phosphate buffered saline (PBS), and lysed in cell lysis buffer containing 50 mM β-glycerophosphate (pH 7.2), 100 *µ*M sodium vanadate, 2 mM MgCl₂, 1mM EGTA Triton X-100 (0.5 percent), leupeptin (2 *µ*g/ml), aprotinin (2 *µ*g/ml), and 1 mM dithiothreitol (600 *µ*l). After centrifugation for 10 minutes at maximum speed in a microfuge, COS cell lysates containing 0.5 to 1 mg of soluble protein were subjected to FPLC on a MONO Q column, and eluted fractions were assayed for MAPK activity. The activation of MAPK occurred in COS cells deprived of serum and in the absence of any added growth factor. The activity of MAPK was similar to that observed after stimulation of control cells with EGF. Stimulation of COS cells transiently overexpressing MEKK with EGF resulted in only a slight increase in MAPK activity compared to that observed with MEKK expression alone.

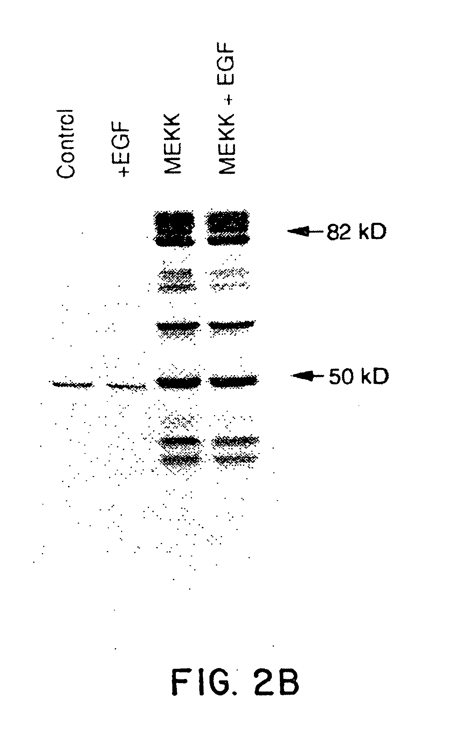

The MEKK protein was detected in transfected COS cells by immunoblotting. Fig. 2B shows an immunoblot showing expression of MEKK in cells transfected with vector only (control) or vector encoding MEKK that were treated with or without EGF. Equal amounts (100 *µ*g) of soluble protein lysate from COS cells were placed on the gel for immunoblotting. Only the 50-kD MEKK immunoreactive fragment was detected in lysates from control COS cells. Transient expression of MEKK in COS cells yielded a predominant 82-kD band that was slightly larger than that observed in PC12, Rat la, or NIH 3T3 cells. This apparently results from the use of a methionine at position 441 rather than 486 for initiation of translation. The bands above the 82kD MEKK band appear to result from phosphorylation of the MEKK protein. The group of bands below the 82-kD MEKK protein are believed to result from proteolysis. Addition of the 15-amino acid MEKK peptide antigen to the antiserum during immunoblotting prevented detection of all of the immunoreactive bands; these bands were not detected in extracts of control COS cells, an indication that they were derived from the expressed MEKK protein.

Expression of MEKK in COS cells was found to activate MEK, the kinase that phosphorylates and activates MAPK. Recombinant MAPK was used to assay MEK activity in COS cell lysates that had been fractionated by fast protein liquid chromatography (FPLC) on a Mono S column. A cDNA encoding p42 MAPK from *Xenopus laevis* was cloned into the pRSETB expression vector. This construct was used for expression in the LysS strain of *Escherichia coli* BL21(DE3) of a p42 MAPK fusion protein containing a polyhistidine sequence at the NH₂-terminus. Cultures containing the expression plasmid were grown at 37°C to an optical density of 0.7 to 0.9 at 600 nM. Isopropyl-*β*-thiogalactopyranoside (0.5 mM) was added to induce fusion protein synthesis and the cultures were incubated for 3 hours. The cells were then collected and lysed by freezing, thawing, and sonication. The lysate was centrifuged at 10,000g for 15 minutes at 4°C. The supernatant was then passed over a Ni²⁺⁻ charged Sepharose resin and the soluble recombinant MAPK was eluted in sodium phosphate buffer (pH 4.5). The purified recombinant MAPK was more than 80 percent pure. The purified recombinant MAPK served as a substrate for MEK and catalyzed the phosphorylation of a peptide consisting of residues 662 to 681 of the EGF receptor (EGFR⁶⁶²⁻⁶⁸¹).

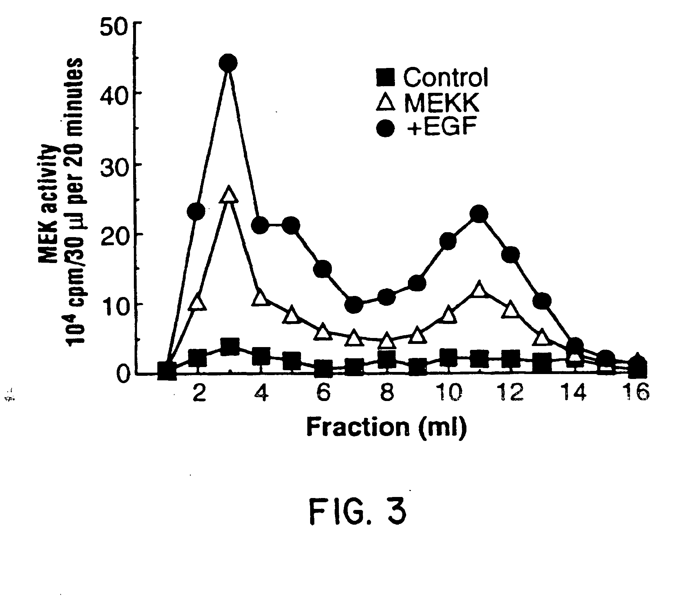

Soluble cell lysates from COS cells transiently transfected with MEKK, mock-transfected (control), or mock-transfected and treated with EGF (30 ng/ml) (+EGF), were fractionated by FPLC on a Mono S column and endogenous MEK activity was measured. Endogenous MAPK eluted in fractions 2 to 4, whereas MEK was contained in fractions 9 to 13. For assaying endogenous MEK activity, cells were washed twice in cold PBS and lysed in 650 *µ*l of a solution containing 50 mM *β*-glycerophosphate, 10 mM 2-*N-*morpholinoethane-sulfonic acid (pH 6.0), 100 *µ*M sodium vanadate, 2 mM MgCl₂, 1 mM EGTA, Triton X-100 (0.5 percent), leupeptin (5 *µ*g/ml), aprotinin (2 *µ*g/ml), and 1 mM dithiothreitol. After centrifugation at maximum speed for 10 minutes in a microfuge, soluble cell lysates (1 to 2 mg of protein) were applied to a Mono S column equilibrated in elution buffer (50 mM *β*-glycerophosphate, 10 mM MES (pH 6.0), 100 *µ*M sodium vanadate, 2 mM MgCl₂, 1 mM EGTA, and 1 mM dithiothreitol. The column was washed with buffer (2 ml) and bound proteins were eluted with a 30ml linear gradient of 0 to 350 mM NaCl in elution buffer. A portion (30 *µ*l) of each fraction was assayed for MEK activity by mixing with buffer (25 mM *β*-glycerophosphate, 40 mM *N*-(2-hydroxyethyl)piperazine-N'-(2-ethanolsulfonic acid) (pH7.2) 50 mM sodium vanadate, 10 mM MgCl₂, 100 *µ*M γ-³²P-ATP (3000 to 4000 cpm/pmol), inhibitor protein-20 (IP-20; TTYADFIASGRTGRRNAIHD; 25 *µ*g/ml), 0.5 mM EGTA, recombinant MAP kinase (7.5 *µ*g/ml), and 200 *µ*M EGFR⁶⁶²⁻⁶⁸¹) in a final volume of 40 *µ*l. After incubation at 30 °C for 20 minutes, the incorporation of γ-³²P-ATP into EGFR⁶⁶²⁻⁶⁸¹ was measured. In this assay, the ability of each column fraction to activate added recombinant MAPK was measured by the incorporation of γ-³²P-ATP into the MAPK substrate, a peptide derived from the EGF receptor (EGFR). Referring to Fig. 3, the first peak of activity eluted represents endogenous activated MAPK, which directly phosphorylates the EGFR peptide substrate.

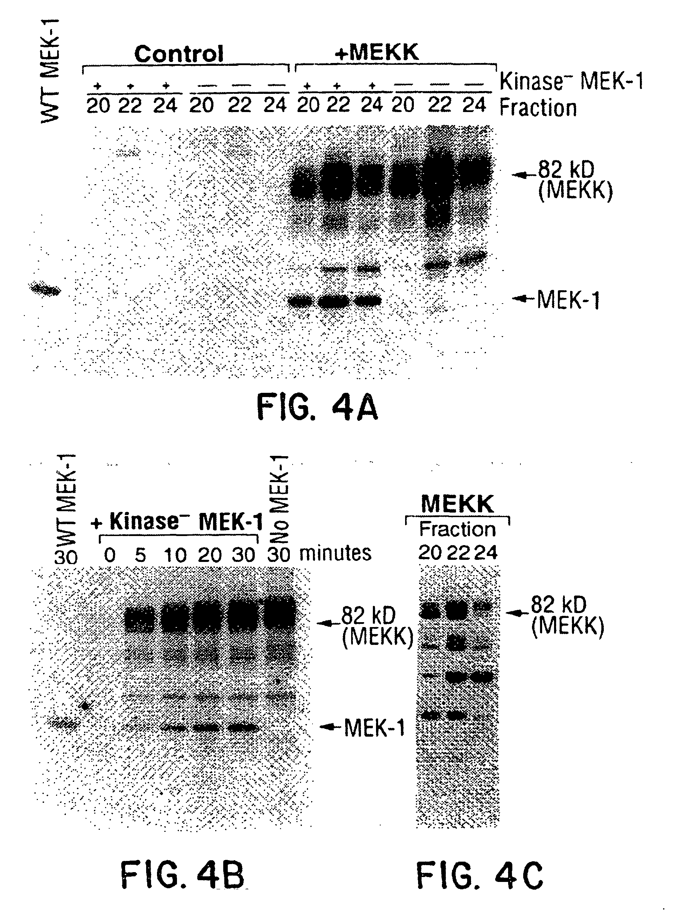

The second peak of activity represents the endogenous MEK in COS cells. Endogenous MEK activity was characterized by fractionation of Mono S FPLC. Fig. 4A indicates that endogenous MEK was activated in cells overexpressing MEKK; the activity of MEK was approximately half of that observed in control cells stimulated with EGF. Thus, expression of MEKK appears to activate MAPK by activating MEK.

COS cell lysates were fractionated by FPLC on a Mono Q column to partially purify the expressed MEKK. Purified recombinant MEK-1 was then used as a substrate for MEKK in the presence of γ-³²P-ATP to determine whether MEKK directly phosphorylates MEK-1.

A cDNA encoding MEK-1 was obtained from mouse B cell cDNA templates with the polymerase chain reaction and oligodeoxynucleotide primers corresponding to portions of the 5' coding region and 3' untranslated region of MEK-1. The catalytically inactive MEK-1 was generated by site-directed mutagenesis of Lys³⁴³ to Met. The wild-type MEK-1 and catalytically inactive MEK-1 proteins were expressed in pRSETA as recombinant fusion proteins containing a polyhistidine sequence at their NH₂-termini.

Lysates from COS cells transfected with MEKK or mock-transfected (control) were subjected to FPLC on a Mono Q column as described above. Portions (20 *µ*l) of fractions containing MEKK were mixed with buffer containing 50 mM β-glycerophosphate (pH 7.2), 100 *µ*M sodium vanadate, 2 mM MgCl₂, 1mM EGTA, 50 *µ*M ATP, IP-20 (50 *µ*g/ml), and 10 *µ*l γ-³²P-ATP in a reaction volume of 40 *µ*l and incubated for 40 minutes in the presence (+) or absence (-) of recombinant, catalytically inactive MEK-1 (150 ng) (kinase-MEK-1) . Reactions were stopped by the addition of 5 x SDS sample buffer (10 *µ*l), 1 x SDS buffer contains 2 percent SDS, 5 percent glycerol, 62.5 mM tris-HCl (pH 6.8), 5 percent β-mercaptoethanol, and 0.001 percent bromophenol blue. The samples were boiled for 3 minutes and subjected to SDS-PAGE and autoradiography. Autophosphorylated recombinant wild-type MEK-1 (WT MEK-1) comigrated with phosphorylated catalytically inactive MEK-1. MEKK was capable of phosphorylating MEK-1. Corresponding fractions of lysates from control cells, however, were not able to phosphorylate MEK-1. A modified form of MEK-1 that is catalytically inactive was used in the phosphorylation assay to ensure that it did not autophosphorylate as does wild-type MEK-1. Phosphorylation of catalytically inactive MEX-1 by MEKK was time dependent (Fig. 4B): MEKK was also phosphorylated. Fraction 22 from FPLC on a Mono Q column (20 *µ*l) was incubated with or without recombinant catalytically inactive MEK-1 (0.15 *µ*g) for the indicated times. Phosphorylation of kinase MEK-1 and MEKK was visable after 5 minutes and maximal after about 20 minutes. The time-dependent increase in MEKK phosphorylation correlated with a decreased mobility of the MEKK protein during SDS-PAGE. Immunoblotting demonstrated that the MEKK protein co-eluted (after FPLC on a Mono Q column) with the peak of activity (fraction 22) that phosphorylated MEK (Fig. 4C). The slowly migrating species of MEKK were also detected by immunoblotting.

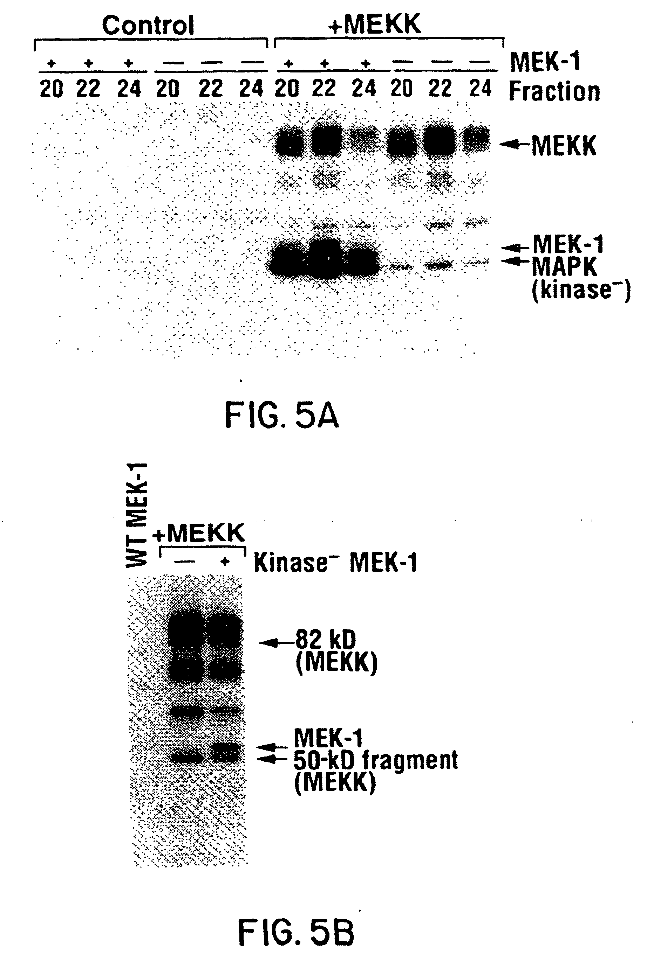

To determine whether the phosphorylation of MEK by overexpressed MEKK resulted in activation of MEK, recombinant wild-type MEK-1 and a modified form of MAPK that is catalytically inactive were used in a coupled assay system. COS cell lysates were separated by Mono Q-FPLC and fractions containing MEKK were assayed for their ability to activate added wild-type MEK-1 such that it would phosphorylate catalytically inactive recombinant MAPK in the presence of γ-³²P-ATP. Lysates from COS cells transfected with MEKK or mock-transfected (control) were fractionated by FPLC on a Mono Q column and portions (20 *µ*l) of fractions containing MEKK were mixed with buffer. Each fraction was incubated in the presence (+) or absence (-) of purified recombinant wild-type MEK-1 (150 ng) and in the presence of purified recombinant, catalytically inactive (kinase) MAPK (300 ng). Referring to Fig. 5A, fractions 20 to 24 from lysates of COS cells transfected with MEKK activated MEK-1. Thus, MEKK phosphorylated and activated MEK-1, leading to MAPK phosphorylation.

To insure that MEKK activated MEK directly, and not through the activation of one or more other kinases contained in the column fractions, overexpressed MEKK was immunoprecipitated from COS cell lysates with an antiserum to COOH-terminal MEKK fusion protein. The *MEKK* cDNA was digested with *Pst* I and *Kpn 1*, thereby creating a 1670-bp fragment that encodes the catalytic domain of MEKK. This fragment was expressed in pRSETC as a recombinant fusion protein containing a polyhistidine sequence at its NH₂₋terminus. The purified COOH-terminal MEKK fusion protein was used to generate polyclonal antisera. Immunoprecipitated MEKK was resuspended in 10 to 15 *µ*l of PAN (10 mM piperazine-N, N'-bis-2-ethanesulfonic acid (Pipes) (pH 7.0), 100 mM NaCl , and aprotinin (20 *µ*g/ml) and incubated with (+) or without (-) catalytically inactive MEK-1 (150 ng) and 25 *µ*Ci of γ-³²P-ATP in 20 mM pipes (pH 7.0), 10 mM MnCl₂, and aprotinin (20 *µ*g/ml ) in a final voume of 20 µl for 15 minutes 30°C. Reactions were stopped by the addition of 5 x SDS sample buffer (5 *µ*l). The samples were boiled for 3 minutes and subjected to SDS-PAGE and autoradiography. Referring to Fig. 5B, MEKK phosphorylated catalytically inactive MEK-1, which comigrated with wild-type MEK-1 on SDS-PAGE. Several phosphorylated bands of overexpressed MEKK were detected in the immunoprecipitates. These bands probably resulted from autophosphorylation of MEKK and corresponded to the forms of MEKK identified by immunoblotting of lysates from COS cells transfected with MEKK (Fig. 1C). Immunoprecipitates obtained with preimmune serum contained no MEKK and did not phosphorylate MEK-1. Thus, MEKK appears to directly phosphorylate MEK, although an associated kinase that co-immunoprecipitates which MEKK cannot be unequivocally excluded.

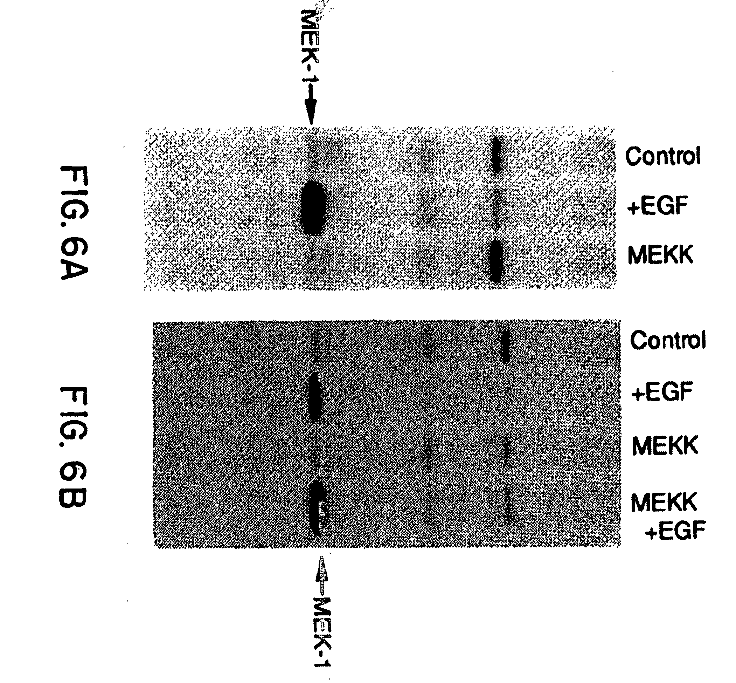

The results show that MEKK can phosphorylate and activate MEK, which in turn phosphorylates and activates MAPK. Raf can also phosphorylate and activate MEK (Fig. 6). COS cells deprived of serum were stimulated with EGF, and Raf was immunoprecipitated with an antibody to the COOH-terminus of Raf-1. Cos cells were transiently transfected with vector alone (control) or with the PCV/M5-MEKK construct (MEKK). Quiescent control cells were treated with or without human EGF (30 ng/ml) for 10 minutes and Raf was immunoprecipitatd from cell lysates with an antibody to a COOH-terminal peptide from Raf. Immunoprecipitated Raf was incubated with catalytically inactive MEK-1 (150 ng) and 25 *µ*l of γ-³²P-ATP. The immunoprecipitated Raf phosphorylated MEK-1 in the presence of γ-³²P-ATP (Fig. 6A). Little or no phosphorylation of MEK-1 by Raf was observed in immunoprecipitates of Raf from COS cells overexpressing MEKK. Treatment of COS cells overexpressing MEKK with EGF resulted in a similar degree of phosphorylation of MEK-1 by immunoprecipitated Raf (Fig. 6B). Cells transfected with MEKK and deprived of serum were treated with EGF, and Raf was immunoprecipitated and incubated with catalytically inactive MEK-1. Equal amounts of Raf were immunoprecipitated in each sample as demonstrated by immunoblotting with antibodies to Raf. The slowest migrating band represents an immunoprecipitated phosphoprotein that is unrelated to Raf or MEK-1. The amount of Raf in the immunoprecipitates from control cells and cells transfected with MEKK was similar as shown by subsequent SDS-PAGE and immunoblotting with the antibody to Raf. Thus, both MEKK and Raf can independently activate MEK.

Identification of the MEKK from mouse further substantiates the conservation in regulation of the MAPK system between yeast and mammals. The results indicate that the mammalian regulatory network controlling MAPK is more complicated than that in yeast. Both MEKK and Raf activate MEK, a convergence point immediately upstream of MAPK for various signals from the cell surface. Raf regulates the MAPK network primarily in response to receptors that have associated tyrosine kinase activity, whereas MEKK might mediate primarily signals originating from receptors that activate G proteins and protein kinase C. This possibility is supported by the findings in specific cell types that down regulation of protein kinase C does not inhibit activation of MAPK in response to growth factors, but does inhibit activation of MAPK in response to agents that stimulate the muscarinic M₁ receptor. The demonstration that Ste20, a protein kinase in *S*. *cerevisiae*, is upstream of Stell in the pheromone-response pathway further supports this hypothesis. In PC12 cells, expression of dominant negative mutant Ras does not inhibit activation of MAPK in response to stimulation of G protein-coupled receptors or activation of protein kinase C, but does inhibit activation of MAPK in response to neuronal growth factor (NGF) or fibroblast growth factor (FGF). It is believed, however, that more complex, cell type specific roles of Raf and MEKK in integrating tyrosine kinase and G protein-couple signaling exist. Defining MEKK and Raf as a divergence in the MAPK network provides a mechanism for differential regulation of this system.

The present invention also includes antibodies capable of selectively binding to an MEKK protein of the present invention. Such an antibody is herein referred to as an anti-MEKK antibody. Polyclonal populations of anti-MEKK antibodies can be contained in an MEKK antiserum. MEKK antiserum can refer to affinity purified polyclonal antibodies, ammonium sulfate cut antiserum or whole atniserum. As used herein, the term "selectively binds to" refers to the ability of such an antibody to preferentially bind to MEKK proteins. Binding can be measured using a variety of methods known to those skilled in the art including immunoblot assays, immunoprecipitation assays, enzyme immunoassays (e.g., ELISA), radioimmunoassays, immunofluorescent antibody assays and immunoelectron microscopy; see, for example, Sambrook et al., *Molecular Cloning: A Laboratory Manual*, Cold Spring Harbor Labs Press, 1989.

Antibodies of the present invention can be either polyclonal or monoclonal antibodies. Antibodies of the present invention include functional equivalents such as antibody fragments and genetically-engineered antibodies, including single chain antibodies, that are capable of selectively binding to at least one of the epitopes of the protein used to obtain the antibodies. Preferably, antibodies are raised in response to proteins that are encoded, at least in part, by a MEKK nucleic acid molecule. More preferably antibodies are raised in response to at least a portion of an MEKK protein and even more preferably antibodies are raised in response to either the amino terminus or the carboxyl terminus of an MEKK protein.

A preferred method to produce antibodies of the present invention includes administering to an animal an effective amount of an MEKK protein to produce the antibody and recovering the antibodies. Antibodies of the present invention have a variety of potential uses that are within the scope of the present invention. For example, such antibodies can be used to identify unique MEKK proteins and recover MEKK proteins.

One embodiment of the present invention includes a nucleic acid molecule encoding the amino terminus of MEKK 1 was cloned into a pRSET bacterial vector to produce an pMEKK-1 recombinant molecule. The pMEKK-1 recombinant molecule was expressed in E. coli and protein encoded by the recombinant molecule was recovered and purified using standard methods known in the art. NZW rabbits were immunized with the purified recombinant MEKK 1 amino terminus protein and antisera was recovered using standard methods known in the art. The foregoing procedure was also performed using a nucleic acid molecule encoding the carboxyl terminus of MEKK 1 protein. The antisera containing anti-MEKK antibody was passed over an affinity column having immobilized MEKK 1 protein bound to Sepharose. The affinity purified anti-MEKK antiserum contained antibodies that selectively bind to MEKK protein from different cell types but did not bind to Raf-1, B-Raf or N-Raf protein.

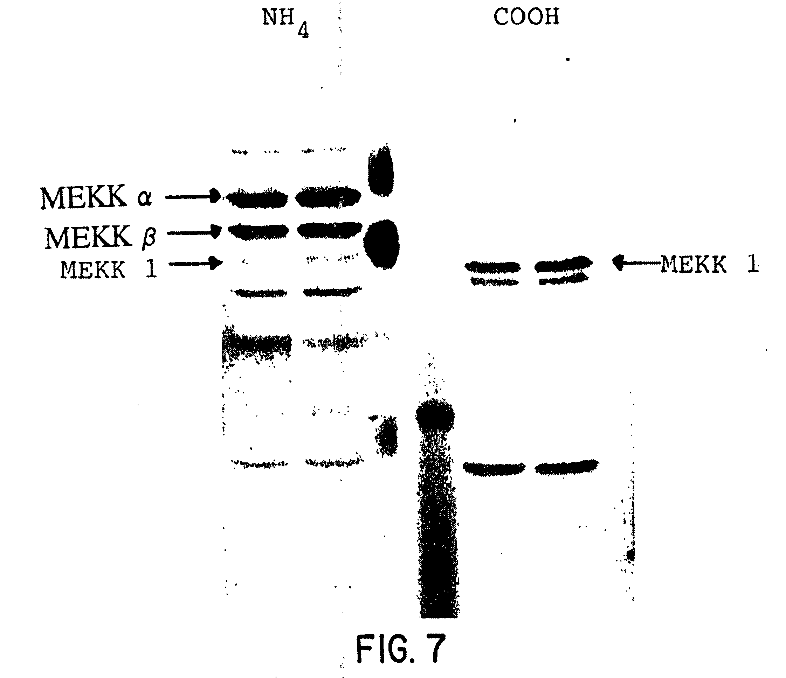

Cells stimulated or not stimulated with EGF were lysed and resolved by SDS PAGE as described above. MEKK proteins contained in the cell lysates were identified by immunoblot using an MEKK antiserum specific for amino terminal region of MEKK 1 and an MEKK antiserum specific for the carboxyl region of MEKK 1. Referring to Fig. 7, MEKK 1 and two higher molecular weight proteins having MEKK activity, MEKK *α* and MEKK *β*, were identified using the MEKK antiserum specific for amino terminal region (NH₄). MEKK 1, and not MEKK *α* and MEKK *β*, was identified using the MEKK antiserum specific for the carboxyl region of MEKK 1 (COOH). Two MEKK immunoreactive species of approximately 95kD and 82kD present in PC12 cell lysates were recognized by an MEKK antisera raised against a portion of the amino terminus of MEKK. Visualization of both of these proteins was inhibited by incubation of the purified recombinant MEKK NH₂-terminal fusion protein antigen with the antibody. A single 95kD MEKK protein was present in MEKK immunoprecipitates, but not in immunoprecipitates using preimmune serum. This 95kD MEKK comigrated with the 95kD MEKK protein present in cell lysates of PC12 cells, and in Ratla, NIH3T3, and Swiss3T3 fibroblasts. More of the 95kD MEKK was expressed in PC12 cells relative to fibroblast cell lines. Immunoblotting with antibodies that specifically recognize Raf-1 or Raf-B indicated that neither of these enzymes were present as contaminants of MEKK immunoprecipitates. 95kD MEKK in MEKK immunoprecipitates did not comigrate with Raf-1 or Raf-B in PC12 cell lysates and no cross-reactivity between MEKK and Raf antibodies was observed.

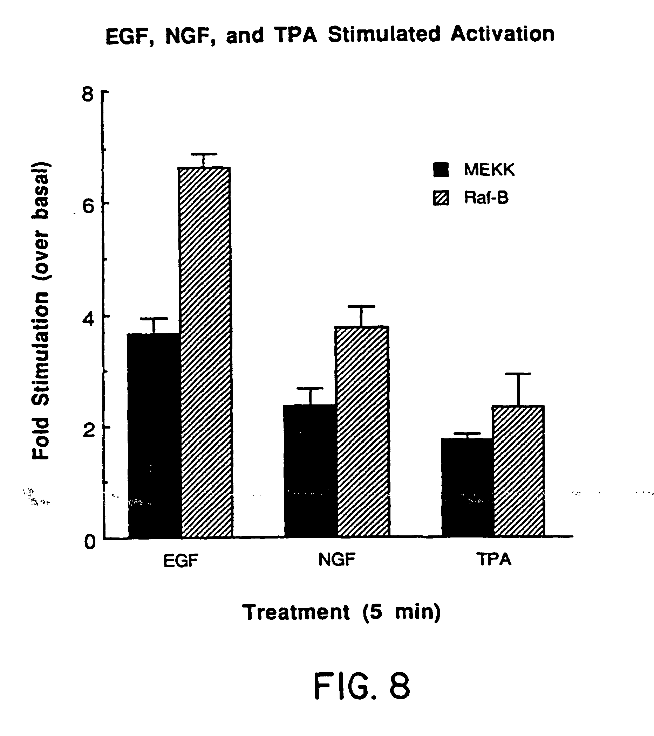

MEKK protein of the present invention can be activated in response to stimulation of cells containing MEKK protein by growth factors. PC12 cells were deprived of serum by incubation in starvation media (DMEM, 0.1% BSA) for 18-20 hours and MEKK was immunoprecipitated from lysates containing equal amounts of protein from untreated controls or cells treated with EGF (30ng/ml) or NGF (100ng/ml) for 5 minutes with the above-described anti-MEKK antibodies speicific for the KH₄-terminal portion of MEKK. Immunoprecipitated MEKK was resuspended in 8*µ*l of PAN [10mM piperazine-N,N'-bis-2-ethanesulfonic acid (Pipes) (pH 7.0), 100mM NaCl, and aprotinin (20*µ*g/ml)] and incubated with catalytically inactive MEK-1 (150ng) and 40*µ*Ci of [γ-³²P]ATP in universal kinase buffer [20mM piperazine-N,N'-bis-2-ethanesulfonic acid (Pipes) (pH 7.0), 10mM MnCl₂, and aprotinin (20*µ*g/ml)] in a final volume of 20*µ*l for 25 minutes at 30°C. Reactions were stopped by the addition of 2X SDS sample buffer (20*µ*l). The samples were boiled for 3 minutes and subjected to SDS-PAGE and autoradiography. Raf-B was immunoprecipitated from the same untreated and treated PC12 cell lysates as above with an antiserum to a COOH-terminal peptide of Raf-B (Santa Cruz Biotechnology, Inc.) and assayed similarly. Raf-1 was immunoprecipitated with an antiserum to the 12 COOH-terminal amino acids of Raf-1 (Santa Cruz Biotechnology, Inc.). Epidermal growth factor (EGF) treatment of serum starved PC12 cells resulted in increased MEKK activity. These results were obtained by measuring the phosphorylation of purified MEK-1 (a kinase inactive form) by immunoprecipitates of MEKK in in vitro kinase assays (Fig. 8). NGF stimulated a slight increase in MEKK activity compared to control immunoprecipitates from untreated cells. Stimulation of MEKK activity by NGF and EGF was similar to Raf-B activation by these agents, although Raf-B exhibited a high basal activity. Activation of c-Raf-1 by NGF and EGF was almost negligible in comparison to that of MEKK or Raf-B.

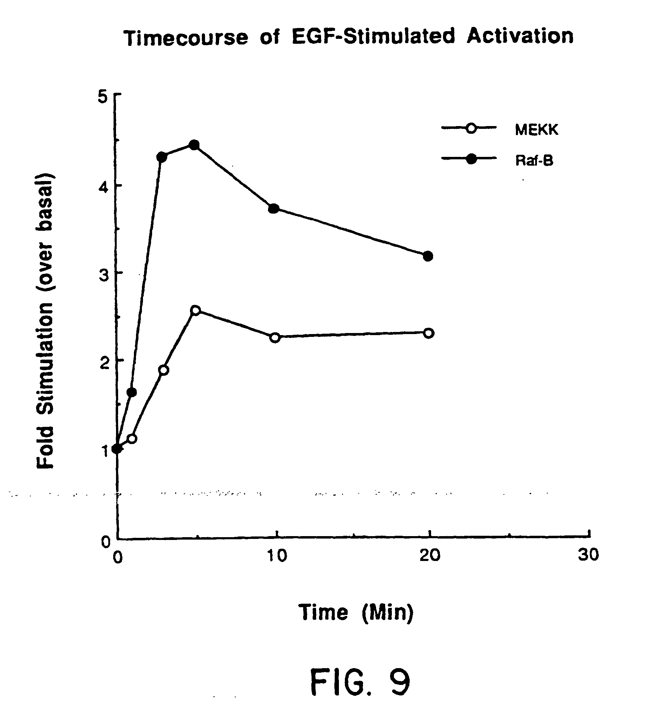

MEKK or Raf-B was immunoprecipitated from lysates of PC12 cells treated with EGF (30ng/ml) for 0, 1, 3, 5, 10, or 20 minutes and incubated with catalytically inactive MEK-1 (150ng) and [γ-³²P]ATP as described above. Data represent the relative magnitude of the response for each timepoint as quantitated by phosphorylimager analysis of radioactive gels from a typical experiment. A timecourse of EGF treatment indicated that MEKK activation reached maximal levels following 5 minutes and persisted for at least 30 minutes (Fig. 9). Raf-B exhibited a similar timecourse; peak activity occurred within 3-5 minutes following EGF treatment and was persistent for up to 20 minutes.

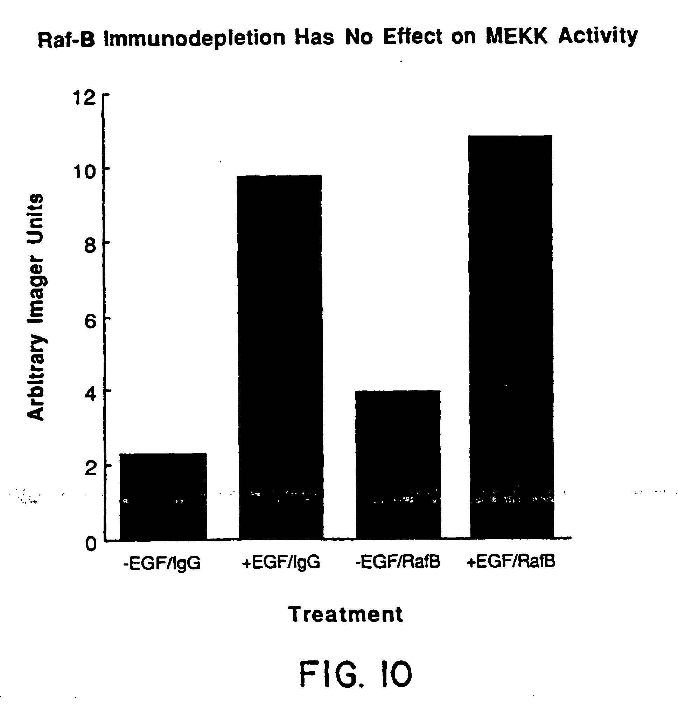

To further dissociate EGF-stimulated MEKK activity from that of Raf-B, Raf-B was immunodepleted from cell lysates prior to MEKK immunoprecipitation. Raf-B was pre-cleared from lysates of serum-starved PC12 cells which had been either treated or not treated with EGF (30ng/ml) for 5 minutes. Raf-B was pre-cleared two times using antisera to Raf-B or using preimmune IgG antisera as a control. The pre-cleared supernatant was then immunoprecipitated with either MEKK or Raf-B antisera and incubated with catalytically inactive MEK-1 and [γ-³²P]ATP as described in detail above. EGF-stimulated and unstimulated PC12 cell lysates were precleared with either IgG or Raf-B antisera and then subjected to immunoprecipitation with MEKK antiserum or Raf-B antibodies. The results shown in Fig. 10 indicate that pre-clearing with Raf-B resulted in a 60% diminution of Raf-B activity as measured by phosphorylimager analysis of Raf-B in vitro kinase assays. EGF-stimulated MEKK activity was unaffected by Raf-B depletion, suggesting that Raf-B is not a component of MEKK immunoprecipitates. At least 40% of the Raf-B activity is resistant to preclearing with Raf-B antibodies. Recombinant wild type MEKK over-expressed in COS cells readily autophosphorylates on serine and threonine residues and the amino-terminus of MEKK is highly serine and threonine rich. MEKK contained in immunoprecipitates of PC12 cells were tested for selective phosphorylation of purified recombinant MEKK amino-terminal fusion protein in in vitro kinase assays.

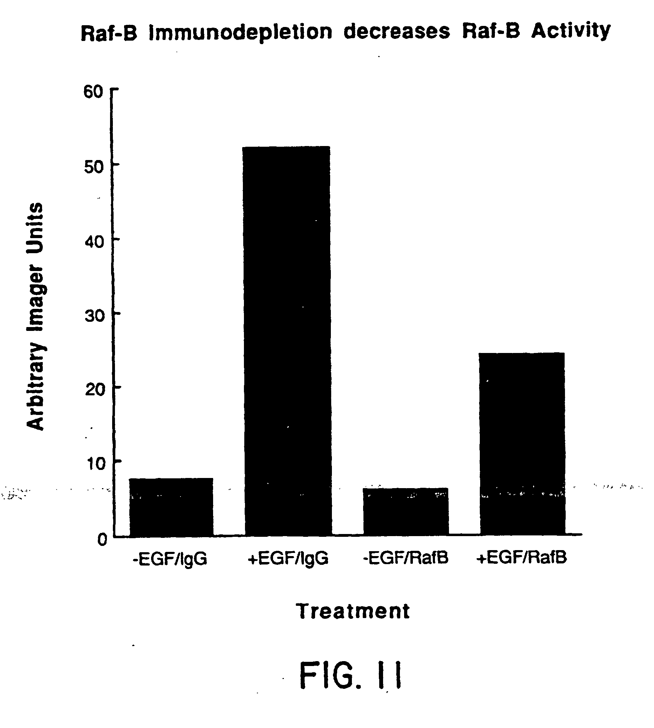

Serum-starved PC12 cells were treated with EGF (30ng/ml) for 5 minutes and equal amounts of protein from the same cell lysates were immunoprecipitated with either MEKK, Raf-B, or preimmune antiserum as a control. Immunoprecipitates were incubated with purified recombinant MEKK NH₂-terminal fusion protein (400ng) and [γ-³²P]ATP as described above. The results shown in Fig. 11 indicate that MEKK immunoprecipitated from lysates of EGF-stimulated and unstimulated PC12 cells robustly phosphorylated the inert 50kD MEKK NH₂-fusion protein, while Raf-B or preimmune immunoprecipitates from EGF-stimulated or unstimulated cells did not use the MEKK NH₂-fusion protein as a substrate. Thus, the EGF-stimulated MEKK activity contained in MEKK immunoprecipites is not due to contaminating Raf kinases.

To insure that the phosphorylation of both MEK-1 and the MEKK NH₂-terminal fusion protein were due to the activity of the 95kD PC12 cell MEKK, cell lysates prepared from EGF-stimulated and unstimulated cells were fractionated by FPLC on a Mono-Q column to partially purify the endogenous MEKK. Lysates from unstimulated control PC12 cells or cells treated with EGF (30ng/ml) for 5 minutes were fractionated by FPLC on a Mono Q column using a linear gradient of 0 to 525mM NaCl. A portion (30*µ*l) of each even numbered fraction was mixed with buffer (20mM piperazine-N,N'-bis-2-ethanesulfonic acid (Pipes) (pH 7.0), 10mM MnCl₂, aprotinin (20*µ*g/ml), 50mM *β*-glycerophosphate (pH 7.2), 1mM EGTA, IP-20 (50*µ*g/ml), 50mM NaF, and 30*µ*Ci [γ-³²P]ATP) containing purified recombinant MEK-1 (150ng) as a substrate in a final volume of 40*µ*l and incubated at 30°C for 25 minutes. Reactions were stopped by the addition of 2X SDS sample buffer (40*µ*l), boiled and subjected to SDS-PAGE and autoradiography. The peak of MEKK activity eluted in fractions 10-12. Portions (30*µ*l) of each even numbered fraction from lysates of EGF-treated PC12 cells were mixed with buffer as described above except containing purified recombinant MEKK NH₂-terminal fusion protein (400ng) as a substrate instead of MEK-1. Purified recombinant kinase inactive MEX-1 or the MEKK NH₂-terminal fusion protein were then used as substrates in the presence of [γ-³²P]ATP to determine whether 95kD MEKK directly phosphorylates either substrate. Fractions 10-14 of lysate from PC12 cells treated with EGF phosphorylated MEK-1 while little MEK-1 phosphorylation occurred in untreated control fractions. The MEKK NH₂-terminal fusion protein was also phosphorylated in the same fractions as was MEK-1, although the peak of activity was slightly broader (fractions 8-16).

Immunoblotting of column fractions demonstrated that the 95kD MEKK protein co-eluted with the peak of activity that phosphorylated either exogenously added kinase inactive MEK-1 or the 50kD MEKK NH₂-terminal fusion protein. Portions (900*µ*l) of even numbered column fractions were concentrated by precipitation with tri-chloroacidic acid and immunoblotted with MEKK antibody. The peak of immunoreactivity eluted in fractions 10-12.

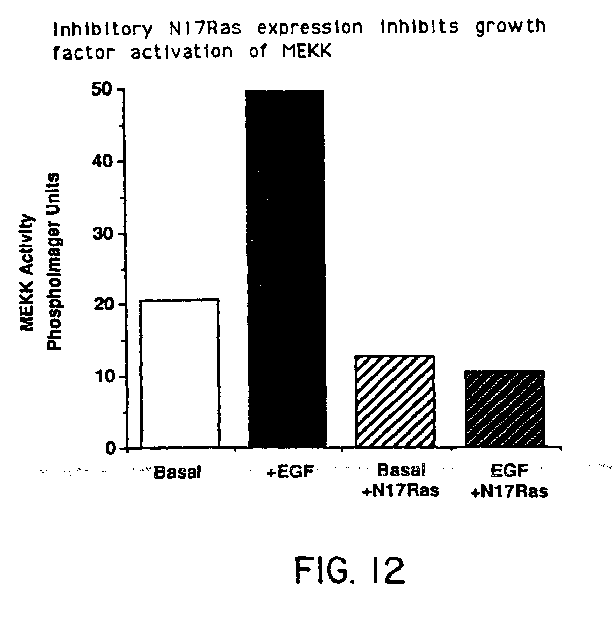

To test whether 95kD PC12 cell MEKK and Raf-B require functional Ras proteins for growth factor mediated signalling, dominant negative Ha-ras(Asn 17) (N¹⁷Ras) was expressed in PC12 cells and EGF-stimulated MEKK or Raf-B activation was assayed in immunoprecipitates using kinase inactive MEK-1 as a substrate. PC12 cells stably expressing dexamethasone inducible N¹⁷Ras were serum starved for 18-20 hours in media containing 0.1% BSA with or without 1*µ*M dexamethasone and then untreated or treated with EGF (30ng/ml) for 5 minutes. Equal amounts of soluble protein from cell lysates was immunoprecipitated with either MEKK or Raf-B antisera and incubated with purified recombinant catalytically inactive MEK-1 and [γ-³²P]ATP as described above. Expression of N¹⁷Ras was induced in PC12 clones stablely transfected with the N¹⁷Ras gene by the addition of dexamethasone to the starvation media. N¹⁷Ras expression inhibited the activation of MEKK by EGF as measured by its ability to phosphorylate kinase inactive MEK-1 (see Fig. 12). EGF-mediated activation of Raf-B was also greatly reduced in N¹⁷Ras expressing PC12 cells compared to uninduced N¹⁷Ras transfectants. Addition of dexamethasone to wild type PC12 cells had no effect on the magnitude of MEKK or Raf-B activation elicited by EGF. PC12 cell clones stablely transfected with the N¹⁷Ras gene are less responsive to EGF-mediated activation of MEKK activity than are wild type PC12 cells. These results indicate that functional Ras is required for growth factor stimulated activation of both Raf-B and MEKK in PC12 cells, suggesting that Ras may mediate its effects on cell growth and differentiation through the activation of multiple protein kinase effectors from both the Raf and MEKK families.

Thus, EGF stimulated a peak of MEKK activity within 5 minutes which persisted for at least 30 minutes following treatment, and was similar to the timecourse of Raf-B activation. Nerve growth factor (NGF) and the phorbol ester TPA also activated MEKK, although to a lesser degree than EGF. MEKK activity in immunoprecipitates or column fractions was dissociable from that of EGF-stimulated c-Raf-1 and Raf-B activities. Forskolin pretreatment abolished both MEKK and Raf-B activation by EGF, NGF, and TPA. Both MEKK and Raf-B activation in response to EGF was inhibited by stable expression of dominant negative N¹⁷ Ras. These findings represent the first demonstration of Ras-dependent MEKK regulation by growth factors and suggest the emergence of a complex intracellular kinase network in which Ras may alternately couple between members of the Raf and MEKK families.

MEKK selectively regulates JUN Kinase activity in a number of cell types (T cells, Neural cells and fibroblasts). Jun Kinase is a distant relative of MAP kinase. The ability of MEKK to activate JUN Kinase is approximately 100 times greater than that for Raf to activate JUN Kinase. JUN Kinase regulates the activity of the transcription factor JUN which is involved in controlling the growth and differentiation of different cell types.

Tumor necrosis factor (TNF) which regulates cell death and other functions in different cell types stimulates MEKK activity. TNF stimulation of MEKK is Raf independent. The signal transduction pathways initiated by UV damage of cells overlaps with the TNF response pathway and also activates MEKK but not Raf. Therefore, both TNF and UV stimulate MEKK activity without activating Raf. Both UV and TNF activation of MEKK is Ras dependent.

Thus, the Ras dependent regulation of MEKK activity in a variety of cell types and shows that Ras controls the activation of the MEKK family in addition to members of the Raf family.

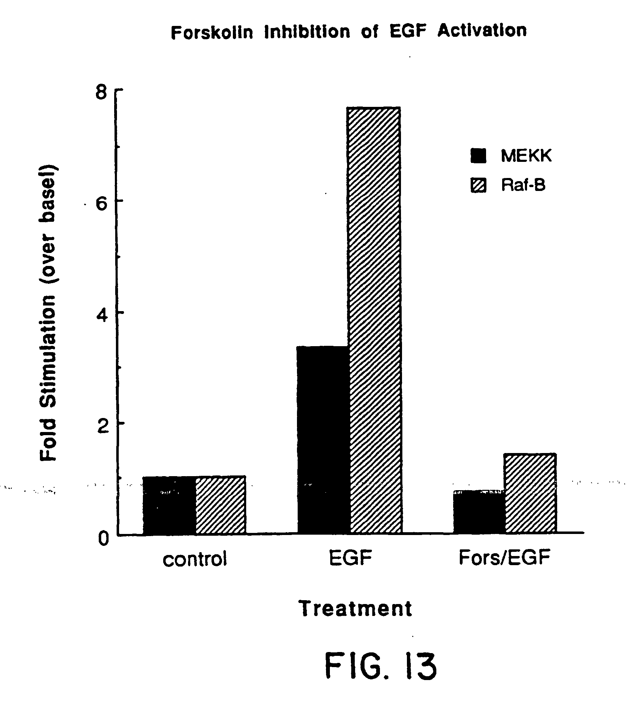

To determine whether the growth factor-mediated activation of 95kD PC12 cell MEKK was inhibited by PKA, forskolin was used to elevate intracellular cAMP and activate PK. Serum-starved PC12 cells were pretreated with or without forskolin (50µM) for 3 minutes to activate protein kinase A and then with EGF (30ng/ml), NGF (100ng/ml), or TPA (200nM) for 5 minutes and MEKK was immunoprecipitated from equal amounts of soluble protein from cell lysates and incubated with purified recombinant catalytically inactive MEK-1 and [γ-³²P]ATP as described above. Raf-B activity was also assayed from the same cell lysates to test whether its regulation differed from that of MEKK. Raf-B was immunoprecipitated from the same cell lysates as described above and assayed for its ability to phosphorylate MEK-1 as described above. Forskolin pretreatment abolished the activation of both MEKK and Raf-B by EGF, NGF, and TPA, as measured by their ability to phosphorylate kinase-inactive MEK-1 (Fig. 13). Forskolin treatment alone had no appreciable effect on either kinase. These results demonstrate that in addition to Raf-1 and Raf-B, PKA activation inhibits growth factor stimulation of 95kD PC12 cell MEKK, suggesting the existence of a common regulatory control point for PKA action which lies between or downstream of Ras and upstream or at the level of each of these three kinases.

To determine if a similar or distinct MEK activity is involved in activation of MAPK though Gᵢ protein coupled receptors, MEK activity in cell lysates from thrombin stimulated Rat la cells was investigated. Thrombin stimulated cells exhibited a MEK activity which co-fractionated with the major MEK peak detected in EGF stimulated cells. The magnitude of MEK activity from thrombin challenged cells was generally two to three-fold less than that observed with EGF stimulation, which correlates with the smaller MAPK response the present inventors have observed in thrombin challenged cells.

Differential regulation of MEK in Rat la and NIH3T3 cells expressing *gip2*, *v-src, v-ras,* or *v-raf* led the present inventor to investigate the protein kinases that are putative regulators of MEK-1. Recently, it was shown that Raf-1 can phosphorylate and activate MEK. Raf activation was assayed in the following manner. Cells were serum starved and challenged in the presence or absence of the appropriate growth factors, as described above. Serum starved Rat la cells were challenged with buffer alone or with EGF and Raf was immunoprecipitated using an antibody recognizing the C terminus of Raf. Cells were lysed by scraping in ice cold RIP buffer (50 mM Tris, pH 7.2, 150 mM NaCl, 0.1% SDS, 0.5% sodium deoxycholate, 1.0% Triton X 100, 10 mM sodium pyrophosphate, 25 mM sodium glycerophosphate, 2mM sodium vanadate, 2.1 *µ*g/ml aprotinin) and were microfuged for 10 min to remove nuclei. The supernatants were normalized for protein content and precleared with protein A Sepharose prior to immunoprecipitation with rabbit antiserum to the C terminus of Raf-1 and protein A Sepharose for 2-3 h at 4°C. The beads were washed twice with ice cold RIPA and twice with PAN (10 mM Pipes, pH 7.0, 100mM NaCl, 21*µ*g/ml aprotinin). A portion of the immunoprecipitate was diluted with SDS sample buffer and used for immunoblot analysis. The remainder was resuspended in kinase buffer (20 mM Pipes pH 7.0, 10 mM MnCl₂, 150 ng kinase-inactive MEK-1, 30 *µ*Ci γ-³²P-ATP and 20 µg/ml aprotinin) in a final volume of 50 µl for 30 min at 30°C. Wild type recombinant MEK-1 was autophosphorylated in parallel as a marker. Reactions were terminated by the addition of 12.5 *µ*l 5X SDS sample buffer, boiled for 5 minutes and subjected to SDS-PAGE and autoradiography.

The immunoprecipitated Raf, in the presence of γ-³²P-ATP, was able to phosphorylate MEK-1. The recombinant MEK-1 used in this assay was kinase inactive to ensure it did not autophosphorylate as is observed with wild type MEK-1. Little or no phosphorylation of MEK-1 by Raf was observed in immunoprecipitates from control cells. EGF challenge clearly stimulated Raf catalyzed phosphorylation of MEK-1; in contrast, thrombin challenge of Rat la cells did not measurably activate Raf even though endogenous MEK was clearly activated. EGF stimulated Raf phosphorylation of recombinant MEK-1 by approximately 2.6-fold over basal. Little phosphorylation of MEK by Raf was observed in Raf immunoprecipitates from Gip2 or v-Src expressing Rat la cells. EGF stimulation was still capable of activating Raf catalyzed phosphorylation of MEK-1 in these cell lines by 1.8 and 1.4-fold, respectively. The blunting of the EFG response in Gip2 and v-Src expressing cells is likely a result of desensitization of the EFG receptor upon constitutive activation of MAPK. The amount of Raf in the immunoprecipitates was shown to be similar by subsequent SDS-PAGE and immunoblotting using Raf antibody. Since thrombin stimulation of MEK is two to three-fold over basal, at least a 1.5-fold stimulation of MEK phosphorylation is expected if Raf significantly contributed to MEK activation by this growth factor. This level of activation was detectable in the EGF stimulated Gip2 and v-Src expressing cells lines. Thus, it is unlikely that the failure to detect thrombin activation of Raf is due to the sensitivity of the assay. Thrombin stimulation of MAPK is maximal at 3 minutes. Stimulation of Rat la cells for 1 or 5 minutes with thrombin did not increase Raf activity.

In NIH3T3 cells, as in Rat la cells, Egf activates Raf approximately 2.7-fold, while thrombin does not. V-Raf expressing NIH3T3 cells showed no increase in MEK-1 phosphorylation. This result was unexpected since MEK was clearly activated in v-Raf expressing NIH3T3 cells. Both the p90 and p75 gag-raf fusion proteins in addition to c-Raf-1 were immunoprecipitated from v-Raf NIH3T3 cells by the antisera. P75gag-raf has been shown to exhibit protein kinase activity, but it is possible that the NH₂ terminal gag fusion protein sterically hinders Raf phosphorylation of recombinant MEK-1 in the *in vitro* assay system. Further studies will have to be done to measure v-Raf kinase activity. The results argue that activation of MEK cannot be accounted for exclusively by the activation of Raf. Additional regulatory kinases for MEK must exist which contribute to MEK activation in thrombin stimulated, Gᵢ protein coupled pathways and in *gip2* and *v-src* transfected cells.

MAPKs are serine/threonine kinases that are thought to be key intermediate molecules in various signal transduction pathways. MAPKs are in turn phosphorylated and activated by MEKs.

Constitutive activation of MEK accounts for most if not all of the increased MAPK activity in oncogene transfected cells. Gip2 and v-Src activated MEK in Rat la cells, while v-Raf most effectively activated MEK in NIH3T3 cells. These oncoproteins activated a similar MEK activity as growth factors. No significant level of MEK stimulation in v-Ras transfected NIH3T3 cells was detected. The findings indicate for a rather tight linkage between MAPK and MEK activation. The present inventor has not observed a significant activation of MAPK independent of MEK activation in oncoprotein expressing growth factor-stimulated cells.

The sensitivity of Raf activation assays involving phosphorylation of kinase inactive MFK-1 suggests that one should be able to easily detect a Raf activity that is 20-25% of that observed with EGF stimulation of both Rat la and NIH3T3 cells. Failure to detect Raf activation in response to thrombin, where both MEK and MAPK activity are easily detected, suggests Raf is not significantly activated by thrombin in Rat la and NIH3T3 cells. This finding suggests an additional protein kinase other than Raf is capable of phosphorylating and activating MEK-1. The present inventor has recently cloned and expressed a mouse MEK kinase (MEKK) that is unrelated to and independent of Raf. Both MEKK and Raf were shown to phosphorylate and activate MEK-1. MEKK is the mouse homolog of the yeast protein kinases Ste 11 and Byr2; MEK is the mouse homolog of the yeast protein kinases Ste7 and Byr1. Raf is unrelated in sequence to Stell and Byr2, suggesting that Raf represents a divergence in mammalian cells from the pheromone responsive protein kinase system defined in yeast.

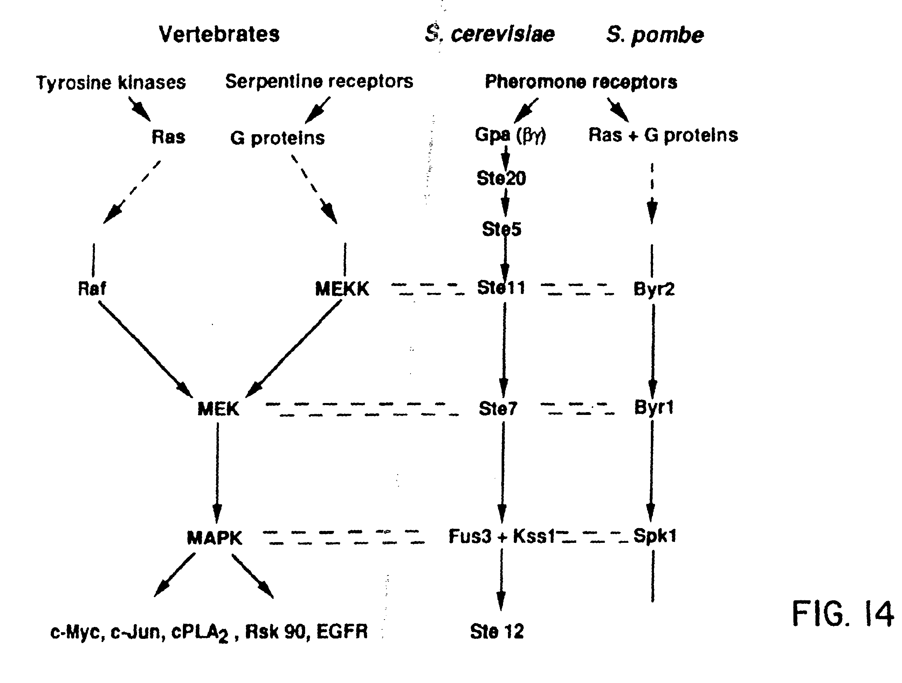

Experiments indicate that Raf and MEKK both activate MEK, which functions as a convergence point for multiple protein kinases that are involved in MAPK activation (See Fig. 14). Thus, Raf and MEKK are protein kinases at the divergence for signal inputs initiated by different cell surface receptors. It is believed that MEKK is a thrombin regulated kinase that regulates MEK in Rat la and NIH3T3 cells. It is also believed that Raf and MEKK are differentially regulated by oncoproteins.

The placement of MEK between MAPK and upstream regulators of MAPK provides integration but also independence of signal networks initiated by growth factor receptors. For example, the serine/threonine kinases Raf and MEKK can phosphorylate MEK, but most likely phosphorylate other proteins as well. MEK is a specialized dual tyrosine/threonine kinase for the selective regulation of MAPK.

One aspect of the present invention relates to the recognition that MEKK activates MAPK. For instance, MAPK is known to be involved in various cellular pathways in mammalian systems. MAPK is known to be involved in cellular mitogenesis, DNA synthesis, cell division and differentiation. MAPK is also recognized as being involved in the activation of oncogenes, such as c-Jun and c-Myc. While not bound by theory, the present inventor believes that MAPK is also intimately involved in various abnormalities having a genetic origin. MAPK is known to cross the nuclear membrane and is believed to be at least partially responsible for regulating the expression of various genes. As such, MAPK is believed to play a significant role in the instigation or progression of cancer, neuronal diseases, autoimmune diseases, allergic reactions, wound healing and inflammatory responses. The present inventor, by being first to identify nucleic acid sequcences encoding MEKK, recognized that it is now possible to regulate the expression of MEKK, and thus regulate the activation of MAPK.

It is also known that MEKK functions in various other capacities besides the activation of MAPK. As such, it is within the scope of the present invention to regulate the expression of MEKK to effect the regulation of other cellular activities that are regulated, either by stimulation or inhibition, by MEKK.

Another aspect of the present invention relates to the recognition that both MEKK as well as Raf are able to activate MAPK. The present inventor has discovered that while MEKK is structurally distinct from Raf, both Raf and MEKK are involved in the regulation of MAPK activity. Therefore, it is within the scope of the present invention to either stimulate or inhibit the expression of Raf and MEKK to achieve desired regulatory results. Thus, in one embodiment, MEKK expression can be inhibited, for example, by use of antisense nucleic acid sequences, in order to block MEKK activity. Alternatively, the expression of Raf can be similarly regulated in the same cell to achieve maximum inhibition of MAPK activity. Regulation of the MAPK system can therefore be fine-tuned by inhibition and/or stimulation of either or both Raf and MEKK expression.

It is within the scope of the present invention to regulate the expression of MEKK through the use of well-known recombinant DNA expression techniques. For example, suitable host cells can be transformed with the MEKK sequence and suitable control elements can be operatively linked with the MEKK sequence to achieve desired expression.

The amount of MEKK enzyme present in the cell can be increased in a variety of ways including, but not limited to, substantially derepressing synthesis of the enzyme, amplifying the copy number of a nucleic acid sequence encoding the enzyme, and combinations thereof.

As used herein, "substantially derepressing synthesis of the enzyme" refers to production of greater amounts of the enzyme than are normally produced by wild-type cells such that the amount and/or rate of substrate-to-product conversion is higher than in wild-type cells (i.e., there is enhanced conversion of substrate to product). Derepression of MEKK enzyme synthesis can be accomplished in a variety of ways, including interfering with the regulatory controls normally exerted over transcription and/or translation of the gene encoding the enzyme and increasing the stability of the messenger RNA (mRNA) corresponding to the enzyme. For example, synthesis of an enzyme which is normally subject to repression, may be increased by at least partially inactivating the respective repressor and/or modifying the operator sequence to reduce the ability of the repressor to bind to it. Modification of transcription (e.g., promoter) and/or translation (e.g., Shine Delgarno sequence) control signals (e.g., initiation, elongation, and/or termination signals) can also enhance both the rate and amount of enzyme production. Methods to derepress enzyme synthesis include recombinant DNA techniques.

As used herein, amplifying the copy number of a nucleic acid sequence in a cell can be accomplished either by increasing the copy number of the nucleic acid sequence in the cell's genome or by introducing additional copies of the nucleic acid sequence into the cell by transformation. Copy number amplification is conducted in a manner such that greater amounts of enzyme are produced, leading to enhanced conversion of substrate to product. For example, recombinant molecules containing nucleic acids of the present invention can be transformed into cells to enhance enzyme synthesis. Transformation can be accomplished using any process by which nucleic acid sequences are inserted into a cell. Transformation techniques include, but are not limited to, transfection, electroporation, microinjection, lipofection, adsorption, and protoplast fusion. After transformation, the cell can produce multiple copies of the nucleic acid sequence, which can either remain on extrachromosomal vectors or be integrated into the host genome. Prior to transformation, the nucleic acid sequence on the recombinant molecule can be manipulated to encode an enzyme having a higher specific activity.

One embodiment of the present invention is a substantially pure MEKK protein. According to the present invention, a substantially pure, or isolated protein, is a protein that has been removed from its natural milieu. As such, "isolated" and "substantially pure" do not necessarily reflect the extent to which the protein has been purified. A substantially pure MEKK protein can be obtained from its natural source. A substantially pure MEKK protein can also be produced using recombinant DNA technology or chemical synthesis. As used herein, a substantially pure MEKK protein can be a full-length MEKK protein or any homologue of such a protein, such as an MEKK protein in which amino acids have been deleted (e.g., a truncated version of the protein, such as a peptide), inserted, inverted, substituted and/or derivatized (e.g., by glycosylation, phosphorylation, acetylation, myristylation, prenylation, palmitation, amidation and/or addition of glycosylphosphatidyl inositol) such that the homologue has kinase activity and/or includes at least one epitope capable of eliciting an immune response against MEKK protein (i.e., when the homologue is administered to an animal as an immunogen, using techniques known to those skilled in the art, the animal will produce a humoral and/or cellular immune response against at least one epitope of MEKK protein). Kinase activity, kinase regulatory activity, as well as the ability of a protein to effect an immune response, can be measured using techniques known to those skilled in the art.

An MEKK protein of the present invention, including a homologue of the full-length protein, has the further characteristic of being encoded by a nucleic acid molecule that is capable of hybridizing, under stringent conditions, with (i.e., to) a nucleic acid comprising at least a portion of the nucleic acid sequence encoding an MEKK protein, such as that disclosed in Table 1. As used herein, the phrase "at least a portion of" an entity refers to an amount of the entity that is at least sufficient to have the functional aspects of that entity. For example, at least a portion of a nucleic acid sequence, as used herein, is an amount of a nucleic acid sequence capable of forming a stable hybrid under stringent hybridization conditions. The nucleic acid sequence represents the deduced sequence of a cDNA (complementary DNA) nucleic acid molecule, the production of which is disclosed below. (It should be noted that since nucleic acid sequencing technology is not entirely error-free, the nucleic acid sequence shown in Table 1, at best, represents an apparent nucleic acid sequence of the nucleic acid molecule encoding at least a portion of an MEKK protein). As used herein, stringent hybridization conditions refer to standard hybridization conditions under which nucleic acid molecules (or sequences), including oligonucleotides, are used to identify similar sequences. Such standard conditions are disclosed, for example, in Sambrook et al., *ibid.* Preferred MEKK proteins of the present invention are encoded by nucleic acid sequences having at least about 80 percent homology (identity within comparable regions) with the nucleic acid sequence shown in Table 1. More preferred MEKK proteins of the present invention are encoded by nucleic acid sequences having at least about 85 percent homology the nucleic acid sequence shown in Table 1, and even more preferred MEKK proteins of the present invention are encoded by nucleic acid sequences having at least about 90 percent homology with the nucleic acid sequence shown in Table 1.

MEKK protein homologues can be the result of natural allelic variation or natural mutation. MEKK protein homologues can also be produced using techniques known in the art including, but not limited to, direct modifications to the protein or modifications to the gene encoding the protein using, for example, classic or recombinant DNA techniques to effect random or targeted mutagenesis. Isolated proteins of the present invention, including homologues, can be identified in a straight-forward manner by the proteins' ability to phosphorylate mammalian MEK protein and/or to elicit an immune response against MEKK proteins. Modifications to MEKK protein of the present invention include truncating the full-length MEKK protein. Preferred modifications to an MEKK protein of the present invention include, for example: deleting at least a portion of a regulatory domain (illustrated in Table 2) to produce a constitutively active MEKK protein; deleting at least a portion of a catalytic domain (illustrated in Table 2) to produce an inactive MEKK protein capable of acting as an inhibitor of proteins capable of regulating MEKK activity; isolating at least a portion of a regulatory domain of an MEKK protein to produce a molecule capable of inhibiting MEKK regulatory proteins; and substituting a lysine residue in the catalytic domain (i.e., phosphotransferase domain) with a methionine residue to inactivate the catalytic domain.

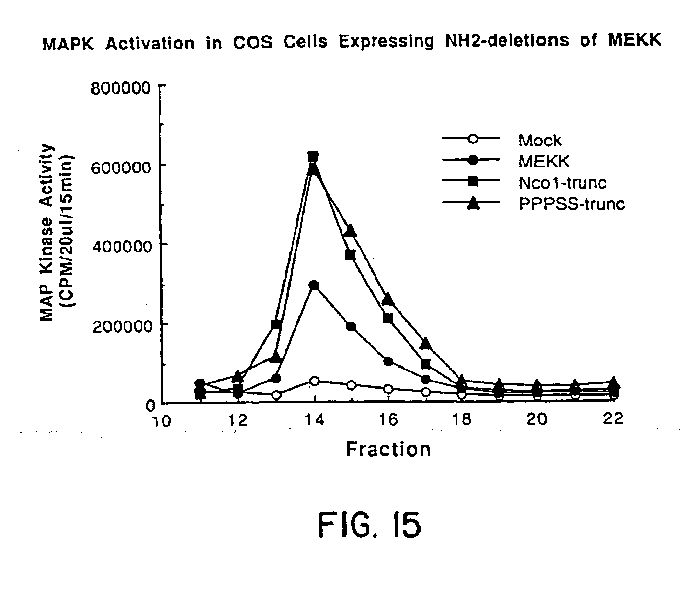

Two truncated forms of MEKK 1 containing the catalytic domain and not the regulatory domain were produced using standard recombinant DNA techniques. Referring to Table 2, a first truncated MEKK molecule was produced by truncating MEKK 1 from residue 1 to residue 215, thereby deleting the PPPSS hinge sequence and the regulatory domain to form the truncated MEKK molecule, PPPSS-trunc. A second smaller truncated MEKK molecule was produced by truncating MEKK 1 from residue 1 to residue 1, thereby removing the PPPSS hinge sequence and the regulatory domain to form the truncated MEKK molecule, Ncol-trunc. The ability of the PPPSS-trunc and Ncol-trunc to activate MAPK activity (determined as described above) was compared with full-length MEKK protein and a negative control protein. The results shown in Fig. 15 indicate that the truncated MEKK molecules were more active than the full-length MEKK. Indeed, the truncated MEKK molecules were at least about 1.5 times more active than full-length MEKK protein. Thus, removal of the regulatory domain of MEKK deregulates the activity of the catalytic domain resulting in improved enzyme activity.

The minimum size of an isolated protein of the present invention is sufficient to form an epitope, a size that is typically at least from about 7 to about 9 amino acids. As is appreciated by those skilled in the art, an epitope can include amino acids that naturally are contiguous to each other as well as amino acids that, due to the tertiary structure of the natural protein, are in sufficiently close proximity to form an epitope.

Functionally equivalent nucleic acid sequences can include nucleic acid sequences containing modifications, such as nucleotide deletions, additions, inversions, and/or substitutions that do not substantially interfere with the nucleic acid sequence's ability to encode a biologically active enzyme. That is, functionally equivalent nucleic acid sequences of the present invention encode enzymes having a biological activity similar to their natural counterparts. Functionally equivalent eukaryotic nucleic acid sequences can also include intervening and/or untranslated sequences surrounding and/or within the coding regions of the nucleic acid sequences.

A functionally equivalent nucleic acid sequence can be obtained using methods known to those skilled in the art (see, for example, Sambrook et al., *ibid.*). For example, nucleic acid sequences can be modified using a variety of techniques including, but not limited to, classic mutagenesis techniques and recombinant DNA techniques, such as site-directed mutagenesis, chemical treatment of a nucleic acid to induce mutations, restriction enzyme cleavage of a nucleic acid fragment, ligation of nucleic acid fragments, polymerase chain reaction (PCR) amplification and/or mutagenesis of selected regions of a nucleic acid sequence, synthesis of oligonucleotide mixtures and ligation of mixture groups to "build" a mixture of nucleic acid sequences, and combinations thereof. Functionally equivalent nucleic acids can be selected from a mixture of modified nucleic acid sequences by screening for the function of the protein encoded by the nucleic acid sequence. A number of screening techniques are known to those skilled in the art including, but not limited to, complementation assays, binding assays, and enzyme assays. Transformation can be accomplished using any process by which nucleic acid sequences are inserted into a cell. Transformation techniques include, but are not limited to, transfection, electroporation, microinjection, lipofection, adsorption, and protoplast fusion. A recombinant cell may remain unicellular or may grow into a tissue or a multicellular organism. Transformed nucleic acid sequences of the present invention can remain extrachromosomal or can integrate into one or more sites within a chromosome of a host cell in such a manner that their ability to be expressed is retained. Integrated nucleic acid sequences often are more stable than extrachromosomal sequences. As such, it is within the scope of the present invention that expression of nucleic acid sequences encoding the enzymes of the present invention may be due to expression of plasmid sequences or to sequences integrated into the host genome.

Preferably, a recombinant cell is produced by transforming a host cell with one or more recombinant molecules, each containing one or more nucleic acid sequences of the present invention operatively linked to an expression vector containing one or more transcription control sequences. A cell can be transformed with one or more recombinant molecules.

As used herein, the phrase "operatively linked" refers to insertion of a nucleic acid sequence into an expression vector in a manner such that the sequence is able to be expressed when transformed into a host cell. As used herein, an expression vector is a DNA or RNA vector that is capable of transforming a host cell, of replicating within the host cell, and of effecting expression of a specified nucleic acid sequence. Expression vectors can be either prokaryotic or eukaryotic, and are typically viruses or plasmids.

Nucleic acid sequences of the present invention can be operatively linked to expression vectors containing regulatory sequences such as promoters, operators, repressors, enhancers, termination sequences, origins of replication, and other regulatory sequences that are compatible with the host cell and that control the expression of the nucleic acid sequences. In particular, expression vectors of the present invention include transcription control sequences. Transcription control sequences are sequences which control the initiation, elongation, and termination of transcription. Particularly important transcription control sequences are those which control transcription initiation, such as promoter, enhancer, operator and repressor sequences. Suitable transcription control sequences include any transcription control sequence that can function in at least one of the host cells of the present invention and can include bacterial, yeast, fungal, insect, animal, and plant transcription control sequences.

A recombinant molecule of the present invention can be any nucleic acid sequence combination herein described operatively linked to any transcription control sequence capable of effectively regulating expression of the nucleic acid sequence in the cell to be transformed.

Recombinant cells of the present invention include any cells transformed with any nucleic acid sequences of the present invention.

It may be appreciated by one skilled in the art that use of recombinant DNA technologies can improve expression of transformed nucleic acid sequences by manipulating, for example, the number of copies of the nucleic acid sequences within a host cell, the efficiency with which those nucleic acid sequences are transcribed, the efficiency with which the resultant transcripts are translated, and the efficiency of post-translational modifications. Recombinant techniques useful for increasing the expression of nucleic acid sequences encoding enzymes include, but are not limited to, operatively linking nucleic acid sequences to high-copy number plasmids, integration of the nucleic acid sequences into one or more host cell chromosomes, addition of vector stability sequences to plasmids, substitutions or modifications of transcription control signals (e.g., promoters, operators, enhancers), substitutions or modifications of translational control signals (e.g., ribosome binding sites, Shine-Delgarno sequences), modification of the nucleic acid sequences encoding enzymes to correspond to the codon usage of the host cell, deletion of sequences that destabilize transcripts, and use of control signals that temporally separate recombinant cell growth from recombinant enzyme production during culturing. The activity of an expressed recombinant enzyme of the present invention may be improved by fragmenting, modifying, or derivatizing nucleic acid sequences encoding enzymes involved in the above-described pathways.

The present invention also includes a method to identify compounds capable of regulating signals initiated from a receptor on the surface of a cell. Such a method comprises the steps of: (a) contacting a cell containing MEKK protein with a putative regulatory compound; (b) contacting the cell with a ligand capable of binding to a receptor on the surface of the cell; and (c) assessing the ability of the putative regulatory compound to regulate cellular signals by determining phosphorylation of the MEKK protein. Step (c) comprises determining the activation of the MEKK protein by measuring the ability of the MEKK protein to phosphorylate MEK protein.

In another embodiment, a method to identify compounds capable of regulating signal transduction in a cell can comprise the steps of: (a) contacting a putative inhibitory compound with MEKK protein to form a reaction mixture; (b) contacting the reaction mixture with MEK protein; and (c) assessing the ability of the putative inhibitory compound to inhibit phosphorylation of the MEK protein by the MEKK protein. The results obtained from step (c) can be compared with the ability of a putative inhibitory compound to inhibit the ability of Raf protein to phosphorylate MEK protein, to determine if the compound can selectively regulate signal transduction involving MEKK protein independent of Raf protein. MEKK, MEK and Raf proteins used in the foregoing methods can be recombinant proteins or naturally-derived proteins.

Another aspect of the present invention includes a kit to identify compounds regulating signals initiated from a receptor on the surface of a cell. Such kits include: (a) at least one cell containing MEKK protein; (b) a ligand capable of binding to a receptor on the surface of the cell; and (c) a means for assessing the ability of a putative regulatory compound to alter phosphorylation of the MEKK protein. Such a means for detecting phosphorylation include methods and reagents known to those of skill in the art, for example, phosphorylation can be detected using phosphocellulose blotting reagents.

In another embodiment, a kit of the present invention can includes: (a) MEKK protein; (b) MEK protein; and (c) a means for assessing the ability of a putative inhibitory compound to inhibit phosphorylation of the MEK protein by the MEKK protein. A kit of the present invention can further comprise Raf protein and a means for detecting the ability of a putative inhibitory compound to inhibit the ability of Raf protein to phosphorylate the MEK protein.

One embodiment of the present invention is a therapeutic composition that, when administered to an animal in an effective manner, is capable of regulating signal transduction in the cells of that animal. In particular, such therapeutic composition is useful for administration to animals to treat medical disorders such as cancer, autoimmune disease, inflammatory responses, allergic responses and neuronal disorders, such as Parkinson's disease and Alzheimer's disease. A therapeutic composition can include at least a portion of an MEKK protein and/or a nucleic acid molecule encoding at least a portion of an MEKK protein of the present invention. A therapeutic composition can include an MEKK protein having kinase activity and/or kinase regulatory activity. Preferably, a therapeutic composition can include an MEKK protein having a kinase domain and/or a kinase regulatory domain.

A therapeutic composition can also include a suitable carrier. As used herein, a "carrier" refers to any substance suitable as a vehicle for delivering a compound of the present invention to a suitable *in vitro* or *in vivo.* site of action. As such, carriers can act as an excipient or formulation of a therapeutic composition containing a compound of the present invention.

## Claims

1. A substantially pure protein selected from the group consisting of: (a) comprising the ammo acid sequence or a kinase catalytic domain or regulatory domain thereof, or
(b) comprising the amino acid sequence or a kinase catalytic domain or regulatory domain thereof.

2. A substantially pure protein comprising an amino acid sequence at least 80% identical to the amino acid sequence or the amino acid sequence

3. The substantially pure protein according to claim 2, wherein the protein has at least 85% identity to said amino acid sequence.

4. The substantially pure protein according to claim 2 or claim 3, wherein the protein has at least 90% identity to said amino acid sequence.

5. A substantially pure protein comprising a kinase domain amino acid sequence at least 80% identical to a kinase domain amino acid sequence or the kinase domain amino acid sequence

6. The substantially pure protein according to claim 5, wherein the protein has at least 85% identity to said kinase domain amino acid sequence.

7. The substantially pure protein according to claim 5 or claim 6, wherein the protein has at least 90% identity to said kinase domain amino acid sequence.

8. An isolated antibody that specifically binds to a protein as claimed in any one of claims 1 to 7.

9. A method to identify compounds capable of regulating signals initiated from a receptor on the surface of a cell comprising:
(a) contacting a cell containing a MEKK protein as defined in claims 1 to 4 with a putative regulatory compound;
(b) contacting said cell with a ligand capable of binding to said receptor and
(c) assessing the ability of said putative regulatory compound to regulate said signals by determining the activity of said MEKK protein.

10. A method to identify compounds capable of regulating signal transduction in a cell comprising:
(a) contacting a putative inhibitory compound with a MEKK protein as defined in claims 1 to 4 to form a reaction mixture,
(b) contacting the reaction mixture with MEK protein; and
(c) assessing the ability of the putative inhibitory compound to inhibit the phosphorylation of said MEK protein by said MEKK protein.

## Patentansprüche

1. Im Wesentlichen reines Protein, das aus der Gruppe ausgewählt wird, bestehend aus (a) umfassend die Aminosäuresequenz oder eine kinase-katalytische Domäne oder regulatorische Domäne davon; oder
(b) umfassend die Aminosäuresequenz oder eine kinase-katalytische Domäne oder regulatorische Domäne davon.

2. Im Wesentlichen reines Protein, das eine Aminosäuresequenz umfasst, die wenigstens zu 80% identisch ist mit der Aminosäuresequenz oder der Aminosäuresequenz

3. Im Wesentlichen reines Protein nach Anspruch 2, wobei das Protein wenigstens 85% Identität zu dieser Aminosäuresequenz aufweist.

4. Im Wesentlichen reines Protein nach Anspruch 2 oder Anspruch 3, wobei das Protein wenigstens 90% Identität zu dieser Aminosäuresequenz aufweist.

5. Im Wesentlichen reines Protein, das eine Kinase-Domäne-Aminosäuresequenz umfasst, die zu wenigstens 80% identisch ist mit der Kinase-Domäne-Aminosäuresequenz oder der Kinase-Domäne-Aminosäuresequenz

6. Im Wesentlichen reines Protein nach Anspruch 5, wobei das Protein wenigstens 85% Identität zu dieser Kinase-Domäne-Aminosäuresequenz aufweist.

7. Im Wesentlichen reines Protein nach Anspruch 5 oder Anspruch 6, wobei das Protein wenigstens 90% Identität zu dieser Kinase-Domäne-Aminosäuresequenz aufweist.

8. Isolierter Antikörper, der spezifisch an ein Protein bindet, das in einem der Ansprüche 1 bis 7 beansprucht wird.

9. Verfahren zur Identifizierung von Verbindungen, die in der Lage sind, Signale, die von einem Rezeptor an der Oberfläche einer Zelle ausgehen, zu regulieren, wobei man:
(a) eine Zelle, die ein in den Ansprüchen 1 bis 4 definiertes MEKK-Protein enthält, mit einer putativen regulatorischen Verbindung in Kontakt bringt;
(b) diese Zelle mit einem Liganden in Kontakt bringt, der in der Lage ist, an diesen Rezeptor zu binden; und
(c) die Fähigkeit dieser putativen regulatorischen Verbindung, diese Signale zu regulieren, durch Bestimmung der Aktivität dieses MEKK-Proteins feststellt.

10. Verfahren zur Identifizierung von Verbindungen, die in der Lage sind, Signal-Transduktion in einer Zelle zu regulieren, wobei man:
(a) eine putative inhibitorische Verbindung mit einem in den Ansprüchen 1 bis 4 definierten MEKK-Protein unter Bildung einer Reaktionsmischung in Kontakt bringt;
(b) die Reaktionsmischung mit MEK-Protein in Kontakt bringt; und
(c) die Fähigkeit dieser putativen inhibitorischen Verbindung, die Phosphorylierung dieses MEK-Proteins durch dieses MEKK-Protein zu inhibieren, feststellt.

## Revendications

1. Protéine sensiblement pure choisie dans le groupe consistant en : (a) comprenant la séquence d'acides aminés : ou un domaine catalytique de kinase ou son domaine régulateur ; ou (b) comprenant la séquence d'acides aminés : ou un domaine catalytique de kinase ou son domaine régulateur.

2. Protéine sensiblement pure comprenant une séquence d'acides aminés identique à au moins 80 % à la séquence d'acides aminés : ou à la séquence d'acides aminés :

3. Protéine sensiblement pure selon la revendication 2, dans laquelle la protéine présente une identité d'au moins 85 % avec ladite séquence d'acides aminés.

4. Protéine sensiblement pure selon la revendication 2 ou la revendication 3, dans laquelle la protéine présente une identité d'au moins 90 % avec ladite séquence d'acides aminés.

5. Protéine sensiblement pure comprenant une séquence d'acides aminés de domaine de kinase identique à au moins 80 % à une séquence d'acides aminés de domaine de kinase : ou à la séquence α acides amines de domaine de kinase :

6. Protéine sensiblement pure selon la revendication 5, dans laquelle la protéine présente une identité d'au moins 85 % avec ladite séquence d'acides aminés de domaine de kinase.

7. Protéine sensiblement pure selon la revendication 5 ou la revendication 6, dans laquelle la protéine présente une identité d'au moins 90 % avec ladite séquence d'acides aminés de domaine de kinase.

8. Anticorps isolé qui se lie spécifiquement à une protéine telle définie dans l'une quelconque des revendications 1 à 7.

9. Procédé pour identifier des composés susceptibles de réguler des signaux initiés par un récepteur à la surface d'une cellule, comprenant :
(a) la mise en contact d'une cellule contenant une protéine MEKK telle que définie dans les revendications 1 à 4 avec un composé régulateur putatif ;
(b) la mise en contact de ladite cellule avec un ligand susceptible de se lier au dit récepteur ; et
(c) l'évaluation de l'aptitude dudit composé régulateur putatif à réguler lesdits signaux par détermination de l'activité de ladite protéine MEKK.

10. Procédé pour identifier des composés susceptibles de réguler la transduction des signaux dans une cellule, comprenant :
(a) la mise en contact d'un composé inhibiteur putatif avec une protéine MEKK telle que définie dans les revendications 1 à 4 pour former un mélange réactionnel ;
(b) la mise en contact du mélange réactionnel avec une protéine MEK ; et
(c) l'évaluation de l'aptitude du composé inhibiteur putatif à inhiber la phosphorylation de ladite protéine MEK par ladite protéine MEKK.


### SEQUENCE LISTING

(1) GENERAL INFORMATION:
   (i) APPLICANT: NATIONAL JEWISH CENTER FOR IMMUNOLOGY AND RESPIRATORY MEDICINE
   (ii) TITLE OF INVENTION: METHOD AND PRODUCT FOR REGULATING CELL RESPONSIVENESS TO EXTERNAL SIGNALS
   (iii) NUMBER OF SEQUENCES: 9
   (iv) CORRESPONDENCE ADDRESS:
      (A) ADDRESSEE: GRAHAM WATT & CO.
      (B) STREET: Riverhead
      (C) CITY: SEVENOAKS
      (D) STATE: Kent
      (E) COUNTRY: GB
      (F) ZIP: TN13 2BN
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: 94 91 5385.2
      (B) FILING DATE: 15-APRIL-1994
   (vii) PRIOR APPLICATION DATA:
      (A) APPLICATION NUMBER: PCT/US94/04178
      (B) FILING DATE: 15-APRIL-1994
   (viii) ATTORNEY/AGENT INFORMATION:
      (A) NAME: HARVEY, David Gareth
      (B) REGISTRATION NUMBER:
      (C) REFERENCE/DOCKET NUMBER: 11478
   (ix) TELECOMMUNICATION INFORMATION:
      (A) TELEPHONE: Int + 44 1732 450055
      (B) TELEFAX: Int + 44 1732 450113
(2) INFORMATION FOR SEQ ID NO: 1:
   (i) SEQUENCE CHARACTERISTICS:
      (A) LENGTH: 3260 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 486..2502
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 672 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Val Thr Ala Val Pro Ala Val Phe Ser Lys Leu Val Thr Met Leu
   1 5 10 15
   Asn Ala Ser Gly Ser Thr His Phe Thr Arg Met Arg Arg Arg Leu Met
   20 25 30
   Ala Ile Ala Asp Glu Val Glu Ile Ala Glu Val Ile Gln Leu Gly Val
   35 40 45
   Glu Asp Thr Val Asp Gly His Gln Asp Ser Leu Gln Ala Val Ala Pro
   50 55 60
   Thr Ser Cys Leu Glu Asn Ser Ser Leu Glu His Thr Val His Arg Glu
   65 70 75 80
   Lys Thr Gly Lys Gly Leu Ser Ala Thr Arg Leu Ser Ala Ser Ser Glu
   85 90 95
   Asp Ile Ser Asp Arg Leu Ala Gly Val Ser Val Gly Leu Pro Ser Ser
   100 105 110
   Thr Thr Thr Glu Gln Pro Lys Pro Ala Val Gln Thr Lys Gly Arg Pro
   115 120 125
   His Ser Gln Cys Leu Asn Ser Ser Pro Leu Ser His Ala Gln Leu Met
   130 135 140
   Phe Pro Ala Pro Ser Ala Pro Cys Ser Ser Ala Pro Ser Val Pro Asp
   145 150 155 160
   Ile Ser Lys His Arg Pro Gln Ala Phe Val Pro Cys Lys Ile Pro Ser
   165 170 175
   Ala Ser Pro Gln Thr Gln Arg Lys Phe Ser Leu Gln Phe Gln Arg Asn
   180 185 190
   Cys Ser Glu His Arg Asp Ser Asp Gln Leu Ser Pro Val Phe Thr Gln
   195 200 205
   Ser Arg Pro Pro Pro Ser Ser Asn Ile His Arg Pro Lys Pro Ser Arg
   210 215 220
   Pro Val Pro Gly Ser Thr Ser Lys Leu Gly Asp Ala Thr Lys Ser Ser
   225 230 235 240
   Met Thr Leu Asp Leu Gly Ser Ala Ser Arg Cys Asp Asp Ser Phe Gly
   245 250 255
   Gly Gly Gly Asn Ser Gly Asn Ala Val Ile Pro Ser Asp Glu Thr Val
   260 265 270
   Phe Thr Pro Val Glu Asp Lys Cys Arg Leu Asp Val Asn Thr Glu Leu
   275 280 285
   Asn Ser Ser Ile Glu Asp Leu Leu Glu Ala Ser Met Pro Ser Ser Asp
   290 295 300
   Thr Thr Val Thr Phe Lys Ser Glu Val Ala Val Leu Ser Pro Glu Lys
   305 310 315 320
   Ala Glu Asn Asp Asp Thr Tyr Lys Asp Asp Val Asn His Asn Gln Lys
   325 330 335
   Cys Lys Glu Lys Met Glu Ala Glu Glu Glu Glu Ala Leu Ala Ile Ala
   340 345 350
   Met Ala Met Ser Ala Ser Gln Asp Ala Leu Pro Ile Val Pro Gln Leu
   355 360 365
   Gln Val Glu Asn Gly Glu Asp Ile Ile Ile Ile Gln Gln Asp Thr Pro
   370 375 380
   Glu Thr Leu Pro Gly His Thr Lys Ala Lys Gln Pro Tyr Arg Glu Asp
   385 390 395 400
   Ala Glu Trp Leu Lys Gly Gln Gln Ile Gly Leu Gly Ala Phe Ser Ser
   405 410 415
   Cys Tyr Gln Ala Gln Asp Val Gly Thr Gly Thr Leu Met Ala Val Lys
   420 425 430
   Gln Val Thr Tyr Val Arg Asn Thr Ser Ser Glu Gln Glu Glu Val Val
   435 440 445
   Glu Ala Leu Arg Glu Glu Ile Arg Met Met Gly His Leu Asn His Pro
   450 455 460
   Asn Ile Ile Arg Met Leu Gly Ala Thr Cys Glu Lys Ser Asn Tyr Asn
   465 470 475 480
   Leu Phe Ile Glu Trp Met Ala Gly Gly Ser Val Ala His Leu Leu Ser
   485 490 495
   Lys Tyr Gly Ala Phe Lys Glu Ser Val Val Ile Asn Tyr Thr Glu Gln
   500 505 510
   Leu Leu Arg Gly Leu Ser Tyr Leu His Glu Asn Gln Ile Ile His Arg
   515 520 525
   Asp Val Lys Gly Ala Asn Leu Leu Ile Asp Ser Thr Gly Gln Arg Leu
   530 535 540
   Arg Ile Ala Asp Phe Gly Ala Ala Ala Arg Leu Ala Ser Lys Gly Thr
   545 550 555 560
   Gly Ala Gly Glu Phe Gln Gly Gln Leu Leu Gly Thr Ile Ala Phe Met
   565 570 575
   Ala Pro Glu Val Leu Arg Gly Gln Gln Tyr Gly Arg Ser Cys Asp Val
   580 585 590
   Trp Ser Val Gly Cys Ala Ile Ile Glu Met Ala Cys Ala Lys Pro Pro
   595 600 605
   Trp Asn Ala Glu Lys His Ser Asn His Leu Ala Leu Ile Phe Lys Ile
   610 615 620
   Ala Ser Ala Thr Thr Ala Pro Ser Ile Pro Ser His Leu Ser Pro Gly
   625 630 635 640
   Leu Arg Asp Val Ala Val Arg Cys Leu Glu Leu Gln Pro Gln Asp Arg
   645 650 655
   Pro Pro Ser Arg Glu Leu Leu Lys His Pro Val Phe Arg Thr Thr Trp
   660 665 670
(2) INFORMATION FOR SEQ ID NO:3:
   (i) SEQUENCE CHARACTERISTICS:
      (A) LENGTH: 671 amino acids
      (B) TYPE: amino acid
      (D) TOPOLOGY: linear
   (ii) MOLECULE TYPE: protein
   (xi) SEQUENCE DESCRIPTION: SEQ ID NO:3:
   Met Val Thr Ala Val Pro Ala Val Phe Ser Lys Leu Val Thr Met Leu
   1 5 10 15
   Asn Ala Ser Gly Ser Thr His Phe Thr Arg Met Arg Arg Arg Leu Met
   20 25 30
   Ala Ile Ala Asp Glu Val Glu Ile Ala Glu Val Ile Gln Leu Gly Val
   35 40 45
   Glu Asp Thr Val Asp Gly His Gln Asp Ser Leu Ala Val Ala Pro Thr
   50 55 60
   Ser Cys Leu Glu Asn Ser Ser Leu Glu His Thr Val His Arg Glu Lys
   65 70 75 80
   Thr Gly Lys Gly Leu Ser Ala Thr Arg Leu Ser Ala Ser Ser Glu Asp
   85 90 95
   Ile Ser Asp Arg Leu Ala Gly Val Ser Val Gly Leu Pro Ser Ser Thr
   100 105 110
   Thr Thr Glu Gln Pro Lys Pro Ala Val Gln Thr Lys Gly Arg Pro His
   115 120 125
   Ser Gln Cys Leu Asn Ser Ser Pro Leu Ser His Ala Gln Leu Met Phe
   130 135 140
   Pro Ala Pro Ser Ala Pro Cys Ser Ser Ala Pro Ser Val Pro Asp Ile
   145 150 155 160
   Ser Lys His Arg Pro Gln Ala Phe Val Pro Cys Lys Ile Pro Ser Ala
   165 170 175
   Ser Pro Gln Thr Gln Arg Lys Phe Ser Leu Gln Phe Gln Arg Asn Cys
   180 185 190
   Ser Glu His Arg Asp Ser Asp Gln Leu Ser Pro Val Phe Thr Gln Ser
           195                 200                 205
   Arg Pro Pro Pro Ser Ser Asn Ile His Arg Pro Lys Pro Ser Arg Pro
       210                 215                 220
   Val Pro Gly Ser Thr Ser Lys Leu Gly Asp Ala Thr Lys Ser Ser Met
   225                 230                 235                 240
   Thr Leu Asp Leu Gly Ser Ala Ser Arg Cys Asp Asp Ser Phe Gly Gly
                   245                 250                 255
   Gly Gly Asn Ser Gly Asn Ala Val Ile Pro Ser Asp Glu Thr Val Phe
               260                 265                 270
   Thr Pro Val Glu Asp Lys Cys Arg Leu Asp Val Asn Thr Glu Leu Asn
           275                 280                 285
   Ser Ser Ile Glu Asp Leu Leu Glu Ala Ser Met Pro Ser Ser Asp Thr
       290                 295                 300
   Thr Val Thr Phe Lys Ser Glu Val Ala Val Leu Ser Pro Glu Lys Ala
   305                 310                 315                 320
   Glu Asn Asp Asp Thr Tyr Lys Asp Asp Val Asn His Asn Gln Lys Cys
                   325                 330                 335
   Lys Glu Lys Met Glu Ala Glu Glu Glu Glu Ala Leu Ala Ile Ala Met
               340                 345                 350
   Ala Met Ser Ala Ser Gln Asp Ala Leu Pro Ile Val Pro Gln Leu Gln
           355                 360                 365
   Val Glu Asn Gly Glu Asp Ile Ile Ile Ile Gln Gln Asp Thr Pro Glu
       370                 375                 380
   Thr Leu Pro Gly His Thr Lys Ala Lys Gln Pro Tyr Arg Glu Asp Ala
   385                 390                 395                 400
   Glu Trp Leu Lys Gly Gln Gln Ile Gly Leu Gly Ala Phe Ser Ser Cys
                   405                 410                 415
   Tyr Gln Ala Gln Asp Val Gly Thr Gly Thr Leu Met Ala Val Lys Gln
               420                 425                 430
   Val Thr Tyr Val Arg Asn Thr Ser Ser Glu Gln Glu Glu Val Val Glu
           435                 440                 445
   Ala Leu Arg Glu Glu Ile Arg Met Met Gly His Leu Asn His Pro Asn
       450                 455                 460
   Ile Ile Arg Met Leu Gly Ala Thr Cys Glu Lys Ser Asn Tyr Asn Leu
   465                 470                 475                 480
   Phe Ile Glu Trp Met Ala Gly Gly Ser Val Ala His Leu Leu Ser Lys
                   485                 490                 495
   Tyr Gly Ala Phe Lys Glu Ser Val Val Ile Asn Tyr Thr Glu Gln Leu
                   500                     505                     510
   Leu Arg Gly Leu Ser Tyr Leu His Glu Asn Gln Ile Ile His Arg Asp
               515                     520                     525
   Val Lys Gly Ala Asn Leu Leu Ile Asp Ser Thr Gly Gln Arg Leu Arg
           530                     535                     540
   Ile Ala Asp Phe Gly Ala Ala Ala Arg Leu Ala Ser Lys Gly Thr Gly
   545                     550                     555                     560
   Ala Gly Glu Phe Gln Gly Gln Leu Leu Gly Thr Ile Ala Phe Met Ala
                       565                     570                     575
   Pro Glu Val Leu Arg Gly Gln Gln Tyr Gly Arg Ser Cys Asp Val Trp
                   580                     585                     590
   Ser Val Gly Cys Ala Ile Ile Glu Met Ala Cys Ala Lys Pro Pro Trp
               595                     600                     605
   Asn Ala Glu Lys His Ser Asn His Leu Ala Leu Ile Phe Lys Ile Ala
           610                     615                     620
   Ser Ala Thr Thr Ala Pro Ser Ile Pro Ser His Leu Ser Pro Gly Leu
   625                     630                     635                     640
   Arg Asp Val Ala Val Arg Cys Leu Glu Leu Gln Pro Gln Asp Arg Pro
                       645                     650                     655
   Pro Ser Arg Glu Leu Leu Lys His Pro Val Phe Arg Thr Thr Trp
                   660                     665                     670
(2) INFORMATION FOR SEQ ID NO:4:
   (i) SEQUENCE CHARACTERISTICS:
      (A) LENGTH: 618 amino acids
      (B) TYPE: amino acid
      (D) TOPOLOGY: linear
   (ii) MOLECULE TYPE: protein
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 626 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Asp Glu Gln Glu Ala Leu Asp Ser Ile Met Lys Asp Leu Val Ala
   1 5 10 15
   Leu Gln Met Ser Arg Arg Thr Arg Leu Ser Gly Tyr Glu Thr Met Lys
   20 25 30
   Asn Lys Asp Thr Gly His Pro Asn Arg Gln Ser Asp Val Arg Ile Lys
   35 40 45
   Phe Glu His Asn Gly Glu Arg Arg Ile Ile Ala Phe Ser Arg Pro Val
   50 55 60
   Arg Tyr Glu Asp Val Glu His Lys Val Thr Thr Val Phe Gly Gln Pro
   65 70 75 80
   Leu Asp Leu His Tyr Met Asn Asn Glu Leu Ser Ile Leu Leu Lys Asn
   85 90 95
   Gln Asp Asp Leu Asp Lys Ala Ile Asp Ile Leu Asp Arg Ser Ser Ser
   100 105 110
   Met Lys Ser Leu Arg Ile Leu Leu Leu Ser Gln Asp Arg Asn His Thr
   115 120 125
   Ser Ser Ser Pro His Ser Gly Val Ser Arg Gln Val Arg Ile Lys Pro
   130 135 140
   Ser Gln Ser Ala Gly Asp Ile Asn Thr Ile Tyr Gln Ala Pro Glu Pro
   145 150 155 160
   Arg Ser Arg His Leu Ser Val Ser Ser Gln Asn Pro Gly Arg Ser Ser
   165 170 175
   Pro Pro Pro Gly Tyr Val Pro Glu Arg Gln Gln His Ile Ala Arg Gln
   180 185 190
   Gly Ser Tyr Thr Ser Ile Asn Ser Glu Gly Glu Phe Ile Pro Glu Thr
   195 200 205
   Ser Glu Gln Cys Met Leu Asp Pro Leu Ser Ser Ala Glu Asn Ser Leu
   210 215 220
   Ser Gly Ser Cys Gln Ser Leu Asp Arg Ser Ala Asp Ser Pro Ser Phe
   225 230 235 240
   Arg Lys Ser Gln Met Ser Arg Ala Arg Ser Phe Pro Asp Asn Arg Glu
   245 250 255
   Cys Ser Asp Lys Arg Glu Thr Gln Leu Tyr Asp Lys Gly Val Lys Gly
   260 265 270
   Gly Thr Tyr Pro Arg Arg Tyr His Val Ser Val His His Lys Asp Tyr
   275 280 285
   Asn Asp Gly Arg Arg Thr Phe Pro Arg Ile Arg Arg His Gln Gly Asn
   290 295 300
   Leu Phe Thr Leu Val Pro Ser Ser Arg Ser Leu Ser Thr Asn Gly Glu
   305 310 315 320
   Asn Met Gly Val Ala Val Gln Tyr Leu Asp Pro Arg Gly Arg Leu Arg
   325 330 335
   Ser Ala Asp Ser Glu Asn Ala Leu Thr Val Gln Glu Arg Asn Val Pro
   340 345 350
   Thr Lys Ser Pro Ser Ala Pro Ile Asn Trp Arg Arg Gly Lys Leu Leu
   355 360 365
   Gly Gln Gly Ala Phe Gly Arg Val Tyr Leu Cys Tyr Asp Val Asp Thr
   370 375 380
   Gly Arg Glu Leu Ala Ser Lys Gln Val Gln Phe Asp Pro Asp Ser Pro
   385 390 395 400
   Glu Thr Ser Lys Glu Val Ser Ala Leu Glu Cys Glu Ile Gln Leu Leu
   405 410 415
   Lys Asn Leu Gln His Glu Arg Ile Val Gln Tyr Tyr Gly Cys Leu Arg
   420 425 430
   Asp Arg Ala Glu Lys Ile Leu Thr Ile Phe Met Glu Tyr Met Pro Gly
   435 440 445
   Gly Ser Val Lys Asp Gln Leu Lys Ala Tyr Gly Ala Leu Thr Glu Ser
   450 455 460
   Val Thr Arg Lys Tyr Thr Arg Gln Ile Leu Glu Gly Met Ser Tyr Leu
   465 470 475 480
   His Ser Asn Met Ile Val His Arg Asp Ile Lys Gly Ala Asn Ile Leu
   485 490 495
   Arg Asp Ser Ala Gly Asn Val Lys Leu Gly Asp Phe Gly Ala Ser Lys
   500 505 510
   Arg Leu Gln Thr Ile Cys Met Ser Gly Thr Gly Ile Arg Ser Val Thr
   515 520 525
   Gly Thr Pro Tyr Trp Met Ser Pro Glu Val Ile Ser Gly Glu Gly Tyr
   530 535 540
   Gly Arg Lys Ala Asp Val Trp Ser Leu Gly Cys Thr Val Val Glu Met
   545 550 555 560
   Leu Thr Glu Lys Pro Pro Trp Ala Glu Tyr Glu Ala Met Ala Ala Ile
   565 570 575
   Phe Lys Ile Ala Thr Gln Pro Thr Asn Pro Gln Leu Pro Ser His Ile
   580 585 590
   Ser Glu His Gly Arg Asp Phe Leu Arg Arg Ile Phe Val Glu Ala Arg
   595 600 605
   Gln Arg Pro Ser Ala Glu Glu Leu Leu Thr His His Phe Ala Gln Leu
   610 615 620
   Val Tyr
   625
(2) INFORMATION FOR SEQ ID NO:6:
   (i) SEQUENCE CHARACTERISTICS:
      (A) LENGTH: 89 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:6:
   Ile Arg Phe Gln Pro Asn Asp His Lys Thr Ile Lys Glu Thr Ala Asp
   1 5 10 15
   Glu Glu Leu Lys Ile Phe Glu Gly Ile Lys His Pro Asn Leu Val Arg
   20 25 30
   Tyr Phe Gly Val Glu Leu His Arg Glu Glu Met Tyr Ile Ile Phe Met
   35 40 45
   Glu Tyr Cys Asp Glu Gly Thr Leu Glu Glu Val Ser Arg Leu Gly Leu
   50 55 60
   Gln Glu His Val Ile Arg Leu Tyr Thr Lys Gln Ile Thr Val Ala Ile
   65 70 75 80
   Asn Val Leu His Glu His Gly Asn Val
   85
(2) INFORMATION FOR SEQ ID NO:7:
   (i) SEQUENCE CHARACTERISTICS:
      (A) LENGTH: 92 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:7:
   Thr Tyr Val Arg Asn Thr Ser Ser Glu Gln Glu Glu Val Val Glu Ala
   1 5 10 15
   Leu Arg Glu Glu Ile Arg Met Met Gly His Leu Asn His Pro Asn Ile
               20                  25                  30
   Ile Arg Met Leu Gly Ala Thr Cys Glu Lys Ser Asn Tyr Asn Leu Phe
           35                  40                  45
   Ile Glu Trp Met Ala Gly Gly Ser Val Ala His Leu Leu Ser Lys Tyr
       50                  55                  60
   Gly Ala Phe Lys Glu Ser Val Val Ile Asn Tyr Thr Glu Gln Leu Leu
   65                  70                  75                  80
   Arg Gly Leu Ser Tyr Leu His Glu Asn Gln Ile Ile
                   85                  90
(2) INFORMATION FOR SEQ ID NO:8:
   (i) SEQUENCE CHARACTERISTICS:
      (A) LENGTH: 95 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:8:
   Gln Val Gln Phe Asn Pro Glu Ser Pro Glu Thr Ser Lys Glu Val Asn
   1               5                   10                  15
   Ala Leu Glu Cys Glu Ile Gln Leu Leu Lys Asn Leu Leu His Glu Arg
               20                  25                  30
   Ile Val Gln Tyr Tyr Gly Cys Leu Arg Asp Pro Gln Glu Lys Thr Leu
           35                  40                  45
   Ser Ile Phe Met Glu Leu Ser Pro Gly Gly Ser Ile Lys Asp Gln Leu
       50                  55                  60
   Lys Ala Tyr Gly Ala Leu Thr Glu Asn Val Thr Arg Lys Tyr Thr Arg
   65                  70                  75                  80
   Gln Ile Leu Glu Gly Val His Tyr Leu His Ser Asn Met Ile Val
                   85                  90                  95
(2) INFORMATION FOR SEQ ID NO:9:
   (i) SEQUENCE CHARACTERISTICS:
      (A) LENGTH: 95 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:9:
   Gln Val Gln Phe Asp Pro Asp Ser Pro Glu Thr Ser Lys Glu Val Ser
   1               5                   10                  15
   Ala Leu Glu Cys Glu Ile Gln Leu Leu Lys Asn Leu Gln His Glu Arg
               20                  25                  30
   Ile Val Gln Tyr Tyr Gly Cys Leu Arg Asp Arg Ala Glu Lys Ile Leu
           35                  40                  45
   Thr Ile Phe Met Glu Tyr Met Pro Gly Gly Ser Val Lys Asp Gln Leu
       50                  55                  60
   Lys Ala Tyr Gly Ala Leu Thr Glu Ser Val Thr Arg Lys Tyr Thr Arg
   65                  70                  75                  80
   Gln Ile Leu Glu Gly Met Ser Tyr Leu His Ser Asn Met Ile Val
                   85                  90                  95